# EUROPEAN PATENT SPECIFICATION

(11) **EP 3 376 226 B1**
(45) Date of publication and mention of the grant of the patent: **13.05.2020**
(21) Application number: 17868496.5
(22) Date of filing: 20.01.2017
(51) Int. Cl.: G01N 33/541, G01N 33/549, G01N 33/563, G01N 33/533, G01N 33/534, G01N 33/535, G01N 33/543

(54) **METHOD AND KIT FOR PREPARING ANTIBODY PAIR AND USE OF KIT, AND SYSTEM FOR PREPARING ANTIBODY PAIR**
VERFAHREN UND KIT ZUR HERSTELLUNG EINES ANTIKÖRPERPAARES UND VERWENDUNG DES KITS SOWIE SYSTEM ZUR HERSTELLUNG EINES ANTIKÖRPERPAARES
PROCÉDÉ ET KIT DE PRÉPARATION DE PAIRE D'ANTICORPS ET UTILISATION DU KIT, ET SYSTÈME DE PRÉPARATION DE PAIRE D'ANTICORPS

(43) Date of publication of application: 19.09.2018
(73) Proprietor: Shenzhen New Industries Biomedical Engineering Co., Ltd., Shenzhen, Guangdong 518122 (CN)
(72) Inventor: RAO, Wei, Shenzhen Guangdong 518122 (CN); ZHANG, Yu, Shenzhen Guangdong 518122 (CN)
(74) Representative: Rapisardi, Mariacristina
(86) International application number: PCT/CN2017/071969
(87) International publication number: WO 2018/133039

(56) References cited:
- WO-A1-2007/053837
- WO-A2-2006/055371
- WO-A2-2007/011746
- WO-A2-2013/177062
- CN-A- 103 926 411
- CN-A- 104 245 960
- CN-A- 104 813 158
- CN-A- 105 683 755
- G WANG ET AL: "Persistent expression of biologically active anti-HER2 antibody by AAVrh.10-mediated gene transfer", CANCER GENE THERAPY, vol. 17, no. 8, 7 May 2010 (2010-05-07), pages 559-570, XP055407360, GB ISSN: 0929-1903, DOI: 10.1038/cgt.2010.11
- KYNER J L ET AL: "Immunoregulation of anti-islet cell antibody in insulin-dependent diabetes: Failure to detect anti-idiotypic antibody following seroconversion", CLINICAL IMMUNOLOGY AND IMMUNOPATHOLOGY, SAN DIEGO, CA, US, vol. 53, no. 2, 1 November 1989 (1989-11-01), pages 321-328, XP022958174, ISSN: 0090-1229, DOI: 10.1016/0090-1229(89)90060-3 [retrieved on 1989-11-01]

## Description

### TECHNICAL FIELD

The present application relates to the technical field of Biomedicine, specifically, relates to a method, for preparing an antibody pair and use of a kit in the preparation of an antibody pair.

### BACKGROUND

The double antibody sandwich method is a technique for quantitative detection of a antigen by using two antibodies binding to the same antigen and is widely used in ELISA, CLIA, RIA and other immunological detection analyses because of its advantages of high sensitivity, strong anti-interference ability, and the like.

In the application process of double antibody sandwich method, on the one hand, the formation of steric hindrance after an antibody binds to an antigen will prevent most of other antibodies binding to the antigen again, and the antibodies that can be successfully paired with each other have stringent requirements on the antibody binding angle and the distance between the identified antigen determinants; on the other hand, each detection system has different requirements on antibody pairs since the solid phases and labels adopted are different, for example, not all antibody pairs that can be used in ELISA can be used in CLIA, which puts forward more requirements on antibody pairs.

Paired antibodies or an antibody pair refers to two identical or different antibodies capable of binding to an antigen simultaneously. At present, the general method of preparing paired antibodies is as the following: 1) preparing a batch of monoclonal antibodies against the target antigen by hybridoma technique, ADLib, or phage antibody library and other methods; 2) obtaining the above antibodies in large quantities by ascites or large-scale cell culture and other approaches; 3) combining all the antibodies in pairs (also their own combinations), one used as a capture antibody immobilized on the ELISA plate, magnetic beads and other solid phase medium, and another one used as a labelled antibody labelled with chromogenic compounds (alkaline phosphatase, horseradish peroxidase or luminol and other substances); 4) detecting the target antigen by the sandwich method, determining whether the pairing is successful according to the signal strength.

This method is also known as the shotgun method in the conventional sense, and has a lot of limiting factors. Firstly, due to manpower limitations, the number of antibodies acquired through step 1) is usually only a few dozens, which greatly limits the range of choices; secondly, the high-sensitivity antibodies obtained from step 1) have a greater possibility of not finding antibody with which they are paired and thus are discarded. Thirdly, the large-scale preparation, purification and labelling of antibodies in the pairing screening process are time-consuming and laborious, and the labelling process may put some influences on the antibodies themselves, thus interfering with judgement.

There are few existing improvements on the method of preparation of paired antibodies. Reference can be made to Chinese patent CN 103884842 A. The improvements comprise: 1) labelling the antibodies to be screened with biotin and chromogenic compounds, respectively, and then coating the avidin and performing pairing detection using sandwich ELISA method; 2) eliminating the interference of the antibody dosage to the detection by the normalization method (i.e., dividing the detection signal by the concentration of the used antibody molecule pair); 3) screening is performed according to the principle that the detection signal of the antibody pair composed of the same antibody molecule is not stronger than the detection signal of the antibody pair composed of a antibody molecule and another antibody molecule. Also, as described in European patent WO 2004025248 A2, paired antibodies are screened by the comparison that the affinity of the complex is greater than the cross-reactivity of the antibodies. All the above methods require the preparation of antibodies in advance, and the effect only relies on the improvement of pairing accuracy.

The present invention relates to a method for preparing an antibody pair, the closest prior art are known from G WANG ET AL: "Persistent expression of biologically active anti-HER2 antibody by AAVrh.10-mediated gene transfer", CANCER GENETHERAPY, vol.17,no.8,7 May 2010 (2010-05-07), pages 559-570, XP055407360 GB ISSN:0929-1903, DOI:10.1038/cgt.2010.11. Relevant technologies are also known from KYNER J L ET AL: "Immunoregulation of anti-islet cell antibody in insulin-dependent diabetes: Failure to detect anti-idiotypic antibody following seroconversion", CLINICAL IMMUNOLOGY AND IMMUNOPATHOLOGY, SAN DIEGO, CA, US, vol. 53, no. 2, 1 November 1989 (1989-11-011, pages 321-328,XP022958174, ISSN:0090-1229, DOI:10.1016/0090-1229(89)90060-3, WO 2013/177062 A2 and WO 2007/011746 A2.

### SUMMARY

The present application aims to provide a method for preparing an antibody pair and use of a kit in the preparation of an antibody pair, so as to solve the technical problems in the prior art that the preparation of antibodies requires many processes like preparation, purification, labelling and others and the workload is too large.

In order to achieve the above objectives, according to one aspect of the present application, provided is a method of preparing an antibody pair. The method comprises using an antigen-binding fragment of an existing antibody as a capture antibody and using an anti-crystallizable fragment antibody as a labelled antibody to directly screen a target antibody which can be paired with the existing antibody from the cell culture supernatant; the antigen-binding fragment of the antibody is Fab' or F(ab)₂, preferably F(ab)₂; and the labelled antibody is an anti-Fc antibody.

Further, the method also comprises: optimizing screened antibodies according to a total antibody content in the cell culture supernatant or a binding strength of a antibody to be screened in the cell culture supernatant to a target antigen, and said cell culture supernatant is from hybridoma cells.

Further, the labelled antibody is labelled with a tracer marker which labels the labelled antibody directly or indirectly; preferably, the tracer marker is selected from at least one of an enzyme label, a fluorescent dye, a chemiluminescent dye,isoluminol and its derivatives and a radioactive label.

Further, optimizing step comprises: a) detecting the screened antibodies that can be paired with the capture antibody in the cell culture supernatant by the sandwich method, and recording the resulting signal as OD₁; b) detecting the total antibody content in the cell culture supernatant, and recording the resulting signal as OD₂; c) detecting the binding strength of the antibody to be screened in the cell culture supernatant to the target antigen, and recording the resulting signal as OD₃; and d) evaluating and optimizing the antibody screened in the step a) based on the OD₁/OD₂ or OD₁/OD₃ ratio.

Further, the step d) comprises: 1) if the cell culture is a single cell strain cell culture, then the antibody with higher OD₁/OD₂ is paired better with the existing antibody; 2) if the cell culture is multiple cell strains cell culture, then the cell culture with higher OD₁/OD₃ is selected to be separated into single cells for continuous culture so as to obtain new cell cultures, and the new cell cultures is further optimized following the step 1).

Further, the step d) specifically comprises: dividing the cell cultures into two groups according to the single colony wells and the multiple colony wells; sorting the group of the single colony wells according to OD₁/OD₂ from high to low, and selecting the first N cell strains, wherein N is 1-20; sorting the group of the multiple colony wells according to OD₁/OD₃ from high to low, and selecting the first M cell wells for subcloning, respectively, and detecting the subcloned cells according to the methods of the step a) and the step b) and selecting N cell strains with higher OD₁/OD₂, wherein M is 1-40.

Further, the step b) specifically comprises: detecting the total antibody content in the cell culture supernatant using an antibody that specifically binds to an antibody conserved region or an immunoglobulin binding protein; preferably, the antibody that specifically binds to an antibody conserved region is selected from the mixture of two or more strains of goat anti-mouse IgM, goat anti-mouse IgA, goat anti-mouse IgD, goat anti-mouse IgG1, goat anti-mouse IgG2a, goat anti-mouse IgG2b, goat anti-mouse IgG3, rabbit anti-mouse IgM, rabbit anti-mouse IgA, rabbit anti-mouse IgD, rabbit anti-mouse IgG1, rabbit anti-mouse IgG2a, rabbit anti-mouse IgG2b and rabbit anti-mouse IgG3, and the immunoglobulin binding protein is a Staphylococcus protein A and/or a Streptococcus G protein.

Further, the step c) specifically comprises: coating the target antigen on a solid phase, and detecting the binding strength of the antibody to be screened in the cell culture supernatant to the target antigen by means of a second antibody.

Further, the existing antibody is any one of the group consisting of an anti-gastrin-17 antibody, an antifolate binding protein antibody, an anti-thyroid peroxidase antibody, an anti-thyroglobulin antibody, an anti-insulin antibody, an anti-ferritin antibody, an serum anti-alpha-fetoprotein antibody, an anti-carcinoembryonic antibody, an anti-prostate specific antigen antibody, an anti-luteinizing hormone antibody, an anti-prolactin antibody, an anti-human chorionic gonadotrnpin antibody, an anti-neuron-specific enolase antibody, an anti-carbohydrate antigen 125 antibody , an anti-carbohydrate antigen 153 antibody, an anti- carbohydrate antigen 199 antibody, an anti-cytokeratin nineteen fragment antibody, an anti-carbohydrate antigen 724 antibody, an anti-carbohydrate antigen 242 antibody, an anti-growth hormone antibody, an anti-myoglobin antibody, an anti-carbohydrate antigen 50 antibody, an anti-C reactive Protein antibody, an anti-corticotropin antibody, an anti-creatine kinase isoenzyme antibody, an anti-Sangtec-100 protein antibody, an anti-laminin antibody, an anti- type IV collagen antibody, an anti-brain natural peptide N-terminal precursor protein antibody, an anti-troponin antibody, an anti-serum parathyroid hormone antibody, an anti- serum calcitonin antibody, an anti-procalcitonin antibody, an anti-prostate acid phosphatase antibody, an anti-osteocalcin antibody, an anti-pregnancy-associated protein A antibody, an anti-pepsinogen I antibody, an anti-pepsinogen II antibody, an anti-insulin-like growth factor antibody or an anti-D-dimer antibody. Further, the method comprises a expansion culture of the cell culture containing the screened antibody and a large-scale preparation and purification of the target antibody.

According to another aspect of the present application, provided is use of a kit in the preparation of an antibody pair, and the kit comprises: a capture antibody, which is an antigen-binding fragment of an existing antibody immobilized on a solid phase medium, the antigen-binding fragment of said existing antibody is Fab' or F(ab)₂; and a labelled antibody, which is an anti-crystallizable fragment antibody: and a target antigen which directly or indirectly binds to a solid phase medium; and an antibody that specifically binds to an antibody conserved region or an immunoglobulin binding protein.

Further, the antigen-binding fragment of said existing antibody is F(ab')₂.

Further, said labelled antibody is labelled with a tracer marker, which directly or indirectly labels said labelled antibody.

Further, said tracer marker is selected from at least one of, an enzyme label, a fluorescent dye, a chemiluminescent dye, isoluminol and its derivatives and a radioactive label.

Further, said antibody that specifically binds to an antibody conserved region is selected from the mixture of two or more strains of goat anti-mouse IgM, goat anti-mouse IgA, goat anti-mouse IgD, goat anti-mouse IgG1, goat anti-mouse IgG2a, goat anti-mouse IgG2b, goat anti-mouse IgG3, rabbit anti-mouse IgM, rabbit anti-mouse IgA, rabbit anti-mouse IgD, rabbit anti-mouse IgG1, rabbit anti-mouse IgG2a_{,} rabbit anti-mouse IgG2b and rabbit anti-mouse IgG3, and said immunoglobulin binding protein is a Staphylococcus protein A and/or a Streptococcus G protein.

Further, said solid phase is an ELISA plate, magnetic beads or colloidal gold.

Further, said existing antibody is any one of the group consisting of an anti-gastrin-17 antibody, an antifolate binding protein antibody, an anti-thyroid peroxidase antibody, an anti-thyroglobulin antibody, an anti-insulin antibody, an anti-ferritin antibody, an anti-alpha-fetoprotein antibody, an anti-carcinoembryonic antibody, an anti-prostate specific antigen antibody, an anti-luteinizing hormone antibody, an anti-prolactin antibody, an anti-human chorionic gonadotrnpin antibody, an anti-neuron-specific enolase antibody, an anti-carbohydrate antigen 125 antibody . an anti-carbohydrate antigen 153 antibody, an anti-carbohydrate antigen 199 antibody, an anti-cytokeratin nineteen fragment antibody, an anti-carbohydrate antigen 724 antibody, an anti-carbohydrate antigen 242 antibody, an anti-growth hormone antibody, an anti-myoglobin antibody, an anti-carbohydrate antigen 50 antibody, an anti-C reactive Protein antibody, an anti-corticotropin antibody, an anti-creatine kinase isoenzyme antibody, an anti-Sangtec-100 protein antibody, an anti-laminin antibody, an anti-type IV collagen antibody, an anti-brain natural peptide N-terminal precursor protein antibody, an anti-troponin antibody, an anti-parathyroid hormone antibody, an anti-calcitonin antibody, an anti-procalcitonin antibody, an anti-prostate acid phosphatase antibody, an anti-osteocalcin antibody, an anti-pregnancy-associated protein A antibody, an anti-pepsinogen I antibody, an anti-pepsinogen II antibody, an anti-insulin-like growth factor antibody or an anti-D-dimer antibody.

Further, said kit further comprising: the reagents used in the expansion culture of cell cultures and the large-scale preparation and purification of the target antibody.

Further, said kit is used in a semi-automatic or full-automatic immunoassay analyzer.

Applying the technical solution of the present application, using an antigen-binding fragment of an existing antibody as a capture antibody and using an anti-crystallizable fragment antibody as a labelled antibody to directly screen a target antibody which can be paired with said existing antibody from cell culture supernatant, without requiring the large-scale preparation, purification and labelling of the antibodies to be screened thereby greatly reducing the workload.

### DESCRIPTION OF THE DRAWINGS

The accompany drawings of the description constituting part of the present application are used to provide a further understanding of the present application; and exemplary embodiments and illustrations thereof of the present application are used to explain the present application, and do not unduly limit the present application. In the drawings:
Figure 1 shows a schematic diagram of screening an antibody that can be paired with a capture antibody from the cell culture supernatant by sandwich method according to an embodiment of the present application;
Figure 2 shows a schematic diagram of quantitatively detecting the total antibody content in the cell culture supernatant using a double antibody sandwich method according to an embodiment of the present application; and
Figure 3 shows a schematic diagram of detecting the binding strength of the antibodies to be screened in the cell culture supernatant to the target antigen using an indirect assay according to an embodiment of the present application.

### DESCRIPTION OF SPECIFIC EMBODIMENTS

It should be noted that, in the case of no conflict, the embodiments of the present application and the features of the embodiments may be combined with each other. The present application will be described in detail below with reference to the accompanying drawings and in combination with the embodiments.

Labelled antibodies: The binding properties of the antibody are detected by virtue of labelling the chromogenic compounds such as alkaline phosphatase, horseradish peroxidase, luminol and the like on the antibody.

The second antibody, that is the so-called secondary antibody, can bind specifically to the primary antibody. When the primary antibody is not conducive to labelling in a complex environment, such as the cell culture supernatant, a secondary antibody may be incorporated to label the chromogenic compounds for detection.

Sandwich method: a method of combined detection of an antigen using an antibody pair, or a method of combined detection of an antibody using an antibody pair.

Indirect method: a method of immobilizing antigen to the solid phase carrier, and being detected with the second antibody.

Subcloning: Cells were dispersed by limiting dilution method into individual cells for culture, thereby purifying the cell strain.

Phage antibody library technology: an antibody preparation technology of taking the full set of genes of antibody variable region by PCR amplification, and expressing and screening the target antibody by means of phage surface display technology.

Screening paired antibodies can only be detected based on the sandwich method. If there are an antibody A and an antigen X to which the antibody A specifically bind, it need screen another antibody B paired with A and binding to the antigen X; then A and B act as a capture antibody and a labelled antibody, respectively. If the sandwich detection of the antigen is possible, the pairing is successful. The present application contemplates to directly screen an antibody paired with A from the cell culture supernatant, i.e., the target antibody B is potentially present in the cell culture supernatant. However, there are the following two problems: ① since the supernatant contains a large amount of impure proteins, the efficiency of pairing will be extremely low, regardless of the cell culture is used as a capture antibody or a labelled antibody; ②A is used as a capture antibody, and then the antibody β-containing cell culture supernatant is added after capturing the antigen, and then the labelled antibody is incorporated to bind to the antibody B, and due to the high degree of homology between antibody A and antibody B (unless the two antibodies are selected from different species, but this is rather rare), the labelled antibody may also bind to antibody A and thus cause false positive.

The inventive idea of the present application is as follows: using the antigen-binding fragment of antibody A as a capture antibody, adding antibody-B-containing cell culture supernatant after capturing antigen X, and then using an anti-crystallizable fragment as a labelled antibody. The labelled antibody binds only to the Fc fragment of the antibody, so it binds only to B to avoid false positive. Therefore, using an antigen-binding fragment of an existing antibody (such as Fab fragment) as a capture antibody and using an anti-crystallizable fragment (such as Fc fragment) antibody as a labelled antibody to directly screen the antibody that can be paired with the existing antibody from the cell culture supernatant. Then, evaluating and optimizing the screened antibody in the above step according to the total antibody content in the cell culture supernatant or the binding strength of the antibody to be screened in the cell culture supernatant to the target antigen.

According to a typical embodiment of the present application, provided is a method of preparing an antibody pair. The method comprises using an antigen-binding fragment of an existing antibody as a capture antibody and using an anti-crystallizable fragment antibody as a labelled antibody to directly screen a target antibody which can be paired with the existing antibody from the cell culture supernatant.

Wherein, "an existing antibody" refers to a prepared antibody to be screened for pairing with it, but not specifically refer to antibodies in the prior art.

Applying the technical solution of the present application, the cell culture supernatant can be directly screened to obtain the target antibody which can be paired with the existing antibody without requiring the large-scale preparation, purification and labelling of the antibodies to be screened using an antigen-binding fragment of the existing antibody as a capture antibody and using an anti-crystallizable fragment antibody as a labelled antibody, thereby greatly reducing the workload.

The sandwich method used for screening antibodies in the present application may be an enzyme-linked immunosorbent sandwich assay, a chemiluminescent immunoassay sandwich method, or other sandwich detection methods.

According to a typical embodiment of the present application, the antigen-binding fragment is a Fab fragment; the labelled antibody is an anti-Fc antibody. Of course, in the present application, the antibody is not limited to an IgG antibody, but may be other types of antibody. Accordingly, the capture antibody is an antigen-binding fragment thereof and the labelled antibody is an anti-crystallizable antibody. The capture antibody includes, but not limited to, Fab' or F(ab)₂, and more preferably F(ab)₂, because F(ab)₂ is more stable than Fab'. The capture antibody can be obtained by digesting intact antibodies with pepsin or papain, or can be obtained by genetic engineering method.

According to a typical embodiment of the present application, the method also comprises: optimizing the screened antibody according to the total antibody content in the cell culture supernatant or a binding strength of an antibody to be screened in the cell culture supernatant to a target antigen, so as to obtain an antibody that paired well with the existing antibody.

According to a typical embodiment of the present application, the labelled antibody is labelled with a tracer marker which labels the labelled antibody directly or indirectly; preferably, the tracer marker includes but not limited to at least one of an enzyme label, a fluorescent dye, a chemiluminescent dye, isoluminol and its derivatives and a radioactive label.

Preferably, preferred steps comprise: a) detecting the screened antibody that can be paired with the capture antibody in the cell culture supernatant by the sandwich method, and recording the resulting signal as OD₁; b) detecting the total antibody content in the cell culture supernatant, and recording the resulting signal as OD₂; c) detecting the binding strength of the antibody to be screened in the cell culture supernatant to the target antigen, and recording the resulting signal as OD₃; and d) evaluating and optimizing the antibody screened in the step a) based on the OD₁/OD₂ or

The inventive idea of the present application is as follows: using the antigen-binding fragment of antibody A as a capture antibody, adding antibody-B-containing cell culture supernatant after capturing antigen X, and then using an anti-crystallizable fragment as a labelled antibody. The labelled antibody binds only to the Fc fragment of the antibody, so it binds only to B to avoid false positive. Therefore, using an antigen-binding fragment of an existing antibody (such as Fab fragment) as a capture antibody and using an anti-crystallizable fragment (such as Fc fragment) antibody as a labelled antibody to directly screen the antibody that can be paired with the existing antibody from the cell culture supernatant. Then, evaluating and optimizing the screened antibody in the above step according to the total antibody content in the cell culture supernatant or the binding strength of the antibody to be screened in the cell culture supernatant to the target antigen.

According to a typical embodiment of the present application, provided is a method of preparing an antibody pair. The method comprises using an antigen-binding fragment of an existing antibody as a capture antibody and using an anti-crystallizable fragment antibody as a labelled antibody to directly screen a target antibody which can be paired with the existing antibody from the cell culture supernatant.

Wherein, "an existing antibody" refers to a prepared antibody to be screened for pairing with it, but not specifically refer to antibodies in the prior art.

Applying the technical solution of the present application, the cell culture supernatant can be directly screened to obtain the target antibody which can be paired with the existing antibody without requiring the large-scale preparation, purification and labelling of the antibodies to be screened using an antigen-binding fragment of the existing antibody as a capture antibody and using an anti-crystallizable fragment antibody as a labelled antibody, thereby greatly reducing the workload.

The sandwich method used for screening antibodies in the present application may be an enzyme-linked immunosorbent sandwich assay, a chemiluminescent immunoassay sandwich method, or other sandwich detection methods.

According to a typical embodiment of the present application, the antigen-binding fragment is a Fab fragment; the labelled antibody is an anti-Fc antibody. Of course, in the present application, the antibody is an IgG antibody, but may be other types of antibody. Accordingly, the capture antibody is an antigen-binding fragment thereof and the labelled antibody is an anti-crystallizable antibody. The capture antibody includes, Fab' or F(ab)₂, and more preferably F(ab)₂, because F(ab)₂ is more stable than Fab'. The capture antibody can be obtained by digesting intact antibodies with pepsin or papain, or can be obtained by genetic engineering method.

According to a typical embodiment of the present application, the method also comprises: optimizing the screened antibody according to the total antibody content in the cell culture supernatant or a binding strength of an antibody to be screened in the cell culture supernatant to a target antigen, so as to obtain an antibody that paired well with the existing antibody.

According to a typical embodiment of the present application, the labelled antibody is labelled with a tracer marker which labels the labelled antibody directly or indirectly; preferably, the tracer marker includes at least one of an enzyme label, a fluorescent dye, a chemiluminescent dye, isoluminol and its derivatives and a radioactive label.

Preferably, preferred steps comprise: a) detecting the screened antibody that can be paired with the capture antibody in the cell culture supernatant by the sandwich method, and recording the resulting signal as OD₁; b) detecting the total antibody content in the cell culture supernatant, and recording the resulting signal as OD₂; c) detecting the binding strength of the antibody to be screened in the cell culture supernatant to the target antigen, and recording the resulting signal as OD₃; and d) evaluating and optimizing the antibody screened in the step a) based on the OD₁/OD₂ or OD₁/OD₃ ratio.

Wherein, in step a), the screened antibody that can be paired with the capture antibody in the cell culture supernatant is detected by the sandwich method, and the detected signal is recorded as OD₁. The positive signal indicates that the cell culture to be detected contains the antibody that can be paired with the existing antibody.

In step b), since the amount of effective antibody in the cell culture supernatant is not equal, the strong signal of the culture medium detected in the step a) may be resulted from the large amount of antibody rather than better pairing effect. Therefore, the present application contemplates to obtain the relative value of the antibody amount in the cell culture supernatant, and believes that the antibody with lower effective total antibody content but higher OD₁ has better pairing effect. In this step, the total antibody content in the cell culture (cell culture solution) supernatant is detected, and the resulting signal is recorded as OD₂.

In the step c), the binding strength of the antibody to be screened in the cell culture supernatant to the target antigen is detected.

In the step d), the antibody screened in the step a) is evaluated or optimized based on the OD₁/OD₂ or OD₁/OD₃ ratio.

According to a typical embodiment of the present application, the step d) comprises: 1) if the cell culture is a single cell strain cell culture, then the antibody with higher OD₁/OD₂ is paired better with the existing antibody; 2) if the cell culture is a multiple cell strains cell culture, then the cell culture with higher OD₁/OD₃ is selected to be separated into single cells for continuous culture so as to obtain new cell cultures, and the new cell cultures is further optimized following the step 1). The higher OD₁/OD₃ is, the signal OD₁ that is detected for the screened antibody is closer to the signal OD₃ that is detected for the binding strength of this antibody to the target antigen, indicating that these two antibodies have less interference from each other and better pairing effect.

Preferably, the step d) specifically comprises: dividing the cell cultures into two groups according to the single colony wells and the multiple colony wells; the group of the single colony wells is sorted according to OD₁/OD₂ from high to low, and the first N cell strains are selected, wherein N is 1-20, preferably 10; the group of the multiple colony wells is sorted according to OD₁/OD₃ from high to low, and the first M cell wells are selected respectively for subcloning, and the subcloned cells are detected according to the methods of the step a) and the step b) and N cell strains with higher OD₁/OD₂ are selected, wherein M is 1-40, preferably 20.

According to a typical embodiment of the present application, the step b) specifically comprises: detecting the total antibody content in the cell culture supernatant using an antibody that specifically binds to an antibody conserved region or an immunoglobulin binding protein; preferably, the antibody that specifically binds to an antibody conserved region includes the mixture of two or more strains of goat anti-mouse IgM, goat anti-mouse IgA, goat anti-mouse IgD, goat anti-mouse IgG1, goat anti-mouse IgG2a, goat anti-mouse IgG2b, goat anti-mouse IgG3, rabbit anti-mouse IgM, rabbit anti-mouse IgA, rabbit anti-mouse IgD, rabbit anti-mouse IgG1, rabbit anti-mouse IgG2a, rabbit anti-mouse IgG2b and rabbit anti-mouse IgG3, and the immunoglobulin binding protein includes a Staphylococcus protein A and/or a Streptococcus G protein. The antibody specifically binding to the antibody conserved region that used in this step is selected from the mixture of two or more strains of the above described antibodies.

According to a typical embodiment of the present application, the step c) utilizes indirect method to detect the binding of only the antibody in the culture and the antigen, and specifically comprises: coating the target antigen on a solid phase, and detecting the binding strength of the antibody in the cell culture supernatant to the target antigen by means of a second antibody. In the detection of the indirect method, only the antibody in the culture binds to the antigen, which is different from the effect of the steric hindrance of another antibody in the sandwich method. Therefore, the signal intensity detected in the indirect method is theoretically higher than that in the sandwich method. When the two values are equivalent, these two antibodies do not affect each other for binding to the antigen, and the pairing effect is the best (in the actual operation, due to the environmental difference of the detection, it is desirable to compare the ratio of value obtained in the sandwich method to that obtained in the indirect method).

According to a typical embodiment of the present application, the existing antibody is any one of the group consisting of an anti-gastrin-17 antibody, an antifolate binding protein antibody, an anti-thyroid peroxidase antibody, an anti-thyroglobulin antibody, an anti-insulin antibody, an anti-ferritin antibody, an anti-alpha-fetoprotein antibody, an anti-carcinoembryonic antibody, an anti-prostate specific antigen antibody, an anti-luteinizing hormone antibody, an anti-prolactin antibody, an anti-human chorionic gonadotrnpin antibody, an anti-neuron-specific enolase antibody, an anti-carbohydrate antigen 125 antibody , an anti-carbohydrate antigen 153 antibody, an anti-carbohydrate antigen 199 antibody, an anti-cytokeratin nineteen fragment antibody, an anti-carbohydrate antigen 724 antibody, an anti-carbohydrate antigen 242 antibody, an anti-growth hormone antibody, an anti-myoglobin antibody, an anti-carbohydrate antigen 50 antibody, an anti-C reactive Protein antibody, an anti-corticotropin antibody, an anti-creatine kinase isoenzyme antibody, an anti-Sangtec-100 protein antibody, an anti-laminin antibody, an anti-type IV collagen antibody, an anti-brain natural peptide N-terminal precursor protein antibody, an anti-troponin antibody, an anti-parathyroid hormone antibody, an anti-calcitonin antibody, an anti-procalcitonin antibody, an anti-prostate acid phosphatase antibody, an anti-osteocalcin antibody, an anti-pregnancy-associated protein A antibody, an anti-pepsinogen I antibody, an anti-pepsinogen II antibody, an anti-insulin-like growth factor antibody or an anti-D-dimer antibody.

According to a typical embodiment of the present application, the method also comprises an expansion culture of the cell culture containing the screened antibody and a large-scale preparation and purification of the target antibody.

According to a typical embodiment of the present application, provided is use of a kit in the preparation of an antibody pair. The kit comprises a capture antibody formed by immobilizing the antigen-binding fragment of an existing antibody on a solid phase medium and an anti-crystallizable fragment antibody used as a labelled antibody, which are used to directly screen a target antibody which can be paired with the existing antibody from the cell culture supernatant.

According to a typical embodiment of the present application, the antigen-binding fragment is a Fab fragment; the labelled antibody is an anti-Fc antibody. Of course, in the present application, the antibody is an Ig antibody, but canbe other types of antibody. Accordingly, the capture antibody is the antigen-binding fragment thereof and the labelled antibody is the anti-crystallizable antibody thereof. Preferably, the capture antibody is Fab' or F(ab)₂, and more preferably F(ab)₂, because F(ab)₂ is more stable than Fab'. The capture antibody can be obtained by digesting intact antibodies with pepsin or papain, or can be obtained by genetic engineering method.

According to a typical embodiment of the present application, the labelled antibody is labelled with a tracer marker which labels the labelled antibody directly or indirectly; preferably, the tracer marker is selected from at least one of an enzyme label, a fluorescent dye, a chemiluminescent dye, isoluminol and its derivatives and a radioactive label.

According to a typical embodiment of the present application, the kit also comprises: an antibody that specifically binds to an antibody conserved region or an immunoglobulin binding protein, used for detecting the total antibody content in the cell culture supernatant: preferably, the antibody that specifically binds to an antibody conserved region includes the mixture of two or more strains of goat anti-mouse IgM, goat anti-mouse IgA, goat anti-mouse IgD, goat anti-mouse IgG1, goat anti-mouse IgG2a, goat anti-mouse IgG2b, goat anti-mouse IgG3, rabbit anti-mouse IgM, rabbit anti-mouse IgA. rabbit anti-mouse IgD, rabbit anti-mouse IgG1, rabbit anti-mouse IgG2a, rabbit anti-mouse IgG2b and rabbit anti-mouse IgG3, and the immunoglobulin binding protein includes a Staphylococcus protein A and/or a Streptococcus G protein.

According to a typical embodiment of the present application, the kit also comprises: a target antigen that directly or indirectly binds to the solid phase medium, for detecting the binding strength of only the antibody in the culture with the antigen by means of a second antibody using the indirect method.

According to a typical embodiment of the present application, the solid phase is an ELISA plate, magnetic beads or colloidal gold.

According to a typical embodiment of the present application, the existing antibody is any one of the group consisting of an anti-gastrin-17 antibody, an anti-folate binding protein antibody, an anti-thyroid peroxidase antibody, an anti-thyroglobulin antibody, an anti-insulin antibody, an anti-ferritin antibody, an serum anti-alpha-fetoprotein antibody, an anti-carcinoembryonic antibody, an anti-prostate specific antigen antibody, an anti-luteinizing hormone antibody, an anti-prolactin antibody, an anti-human chorionic gonadotrnpin antibody, an anti-neuron-specific enolase antibody, an anti-carbohydrate antigen 125 antibody, an anti-carbohydrate antigen 153 antibody, an anti-carbohydrate antigen 199 antibody, an anti-cytokeratin nineteen fragment antibody, an anti-carbohydrate antigen 724 antibody, an anti-carbohydrate antigen 242 antibody, an anti-growth hormone antibody, an anti-myoglobin antibody, an anti-carbohydrate antigen 50 antibody, an anti-C reactive Protein antibody, an anti-corticotropin antibody, an anti-creatine kinase isoenzyme antibody, an anti-Sangtec-100 protein antibody, an anti-laminin antibody, an anti- type IV collagen antibody, an anti-brain natural peptide N-terminal precursor protein antibody, an anti-troponin antibody, an anti-serum parathyroid hormone antibody, an anti- serum calcitonin antibody, an anti-procalcitonin antibody, an anti-prostate acid phosphatase antibody, an anti-osteocalcin antibody, an anti-pregnancy-associated protein A antibody, an anti-pepsinogen I antibody, an anti-pepsinogen II antibody, an anti-insulin-like growth factor antibody or an anti-D-dimer antibody.

According to a typical embodiment of the present application, the kit also comprises: the reagents used in an expansion culture of cell cultures and a large-scale preparation and purification of the target antibody.

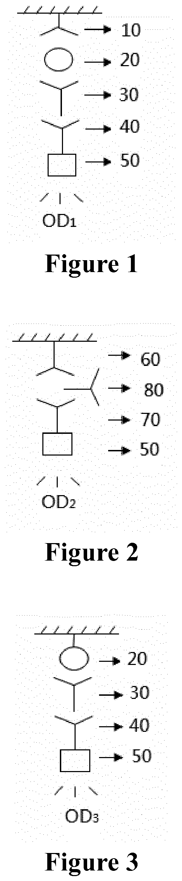

Specifically, in a typical embodiment of the present application, a method of efficiently preparing an antibody pair using a Fab fragment of a high-sensitive antibody as a capture antibody and an anti-Fc antibody as a labelled antibody is as the following steps:
a) As shown in Fig. 1, using the Fab fragment of the existing high-sensitivity antibody as a capture antibody 10, and using the anti-Fc antibody as a labelled antibody 40 (carrying a label 50), from the cell culture supernatant directly screen the antibody 30 (20 is the antigen) that can be paired with the capture antibody by the sandwich method, and the signal detected by the sandwich method is recorded as OD₁;
b) As shown in Fig. 2, the total antibody content in the cell culture supernatant is quantitatively detected using a double antibody sandwich method, that is the total antibody 80 content in the cell culture supernatant is detected by the sandwich method using two antibodies that specifically bind to the antibody conserved region (the second antibody A 60 and the second antibody B 70) and the resulting signal is recorded as OD₂;
incubated at room temperature for 10 min, and 50 µL of 2 M H₂SO₄ was added to terminate the reaction. The absorbance at a wavelength of 450 nm was measured.

### Coating effect of Fab' and F(ab)₂

The anti-TPO antibody T-1 and Fab' and F(ab)₂ thereof were used to coat the ELISA plates to examine their pairing with T-2 antibody. The sandwich ELISA was used to detect the TPO antigens with different concentrations. The results are shown in the below Table 1 (detection of the pairing of T-1 and Fab' and F(ab)₂ thereof with T-2):

**Table 1**

| | 0.01 µg/mL | 0.1 µg/mL | 1 µg/mL |
|---|---|---|---|
| Fab' | 1.96 | 2.65 | 2.74 |
| F(ab)₂ | 2.98 | 3.42 | 3.67 |
| T-1 | 3.09 | 3.64 | 3.78 |

| | | | |
|---|---|---|---|
| Note: The data in the table indicate OD₄₅₀, and the detection background value (under the same condition other than the absence of antigen) is less than 0.2. | | | |

The anti-TPO antibody T-1 and Fab' and F(ab)₂ thereof were used to coat the ELISA plates. Used goat anti-mouse IgG Fc antibody as the second antibody in ELISA, and examined the pairing of three form of T-1 antibody with the T-2 antibody in the cell culture supernatant. The sandwich ELISA was used to detect the TPO antigens with different concentrations. The results are shown in the below Table 2 (detection of the pairing of T-1 and Fab' and F(ab)₂ thereof with T-2 in the cell culture supernatant):

**Table 2**

| | 0.01 µg/mL | 0.1 µg/mL | 1 µg/mL |
|---|---|---|---|
| Fab' | 1.46 | 2.33 | 2.65 |
| F(ab)₂ | 2.45 | 3.02 | 3.64 |
| T-1 | 3.85 | 3.79 | 3.83 |

| | | | |
|---|---|---|---|
| Note: The data in the table indicate OD₄₅₀, and the detection background value (under the same condition other than the absence of antigen) is less than 0.2. | | | |

### Example 2

### Preparation of anti-gastrin-17 antibody pair

Gastrin-17 (G17) is a polypeptide with 17-amino acid and is of great diagnostic significance for tumours and gastric diseases. In the art the double antibody sandwich method is recommended, but this molecule is too small, the preparation of an antibody pair has relatively high difficulty. In the previous studies, the inventors of the present application acquired a mouse-derived monoclonal antibody G-1 having an extremely high affinity with G17. In the present example, the paired antibodies of G-1 (IgG subtype) was prepared.

### 1) Acquisition of F(ab)₂

Reference is made to the method provided by Short Protocols in Immunology (John E. Coligan, USA, 2009) to use the pepsin digestion. Specifically, 2 mL of 3 mg/mL purified G-1 was dialyzed against 200 mL of acetic acid buffer (pH 4.0) at 4°C for 4 h, the concentration was re-measured and adjusted to 2 mg/mL using acetate buffer with the same pH. The pepsin concentration of 0.1 mg/mL was formulated with the acetate buffer (pH 4.0), and 1 mL of the solution was added to the above described antibody solution to make the enzyme to antibody of 1 : 20 to react at 37°C for 4 h. The reaction was terminated by adding 100 µL of 2 mol/L Tris base. The mixture was transferred to a dialysis bag and dialyzed against 1 L PBS of pH 8.0 at 4°C. The dialysate was loaded onto a 5 mm × 100 mm protein A cross-linked agarose gel CL-4B chromatographic column and the unbound effluent is collected. The effluent was concentrated to 4 mL and loaded onto a 26 mm × 900 mm polyacrylamide dextran S-200 Superfine chromatographic column to collect the component with the molecular weight of 110 kDa. The above described product was identified using 10% SDS non-reducing polyacrylamide gel electrophoresis, showing a band only at 110 kDa, a single band at 25 kDa was shown using non-reducing polyacrylamide gel electrophoresis. A280 was detected to determine the final concentration of F(ab)₂.

### 2) Screening the antibodies that can be paired with G-1 by the sandwich ELISA method

The mature Balb/c mice immunized with G17-KLH were challenged by intraperitoneal injection of 300 µg of G17-KLH conjugate three days in advance. The spleen cells were harvested and ground to obtain lymphocyte, and the cell fusion of the lymphocyte and mice myeloma cell sp2/0 was facilitated by using PEG1500. Put them in 20 pieces of 96-well cell culture plates after fusion. Cultured at 37°C, 5% CO₂ for 7 days, replaced the fresh medium (80% RPMI1640, 20% fetal bovine serum) and cultured for another day.

The G-1 F(ab)₂ solution was diluted to 1 µg/mL with a pH 9.6 carbonate buffer and was added to 20 pieces of 96-well ELISA plates at 100 µl/well, incubated at 4°C for 12 h, washed with PBST (containing 0.05% Tween-20 in PBS buffer, pH 7.4). Added 200 µl of 1% OVA (ovalbumin) to incubate at 37°C for 2 h to block the un-reacted site on the ELISAplate and washed once with PBST. 50 µL of 1 ng/mL G17 antigen and 50 µL of cell culture solution were added to each well, incubated at 37°C for 1 h and washed three times with PBST. Added 100 µL of 0.1 µg/mL HRP-labelled goat anti-mouse IgG Fc antibody, incubated at 37°C for 1 h, and washed three times with PBST. Added 100 µL TMB substrate and incubated at room temperature for 10 min. Added 50 µL of 2 M H₂SO₄ to terminate the reaction. The absorbance at a wavelength of 450 nm was measured and recorded as OD₁. Cell cultures with OD₁ > 1.5 were subjected to the detection of the next two steps.

### 3) Detecting the relative amounts of total antibodies in the cell culture supernatant by the double antibody sandwich ELISA method

The mixture of goat anti-mouse IgG1, IgG2a, IgG2b, IgG3 antibody (no cross-over from each other) was diluted to 2 µg/mL with a pH 9.6 carbonate buffer. Added them to ELISA plates at 100 µl/well, incubated at 4°C for 12 h, washed with PBST (containing 0.05% Tween-20 in PBS buffer, pH 7.4). Added 200 µL of 1% OVA (ovalbumin), incubated at 37°C for 2 h, and washed once with PBST. 100 µL of the cell culture solution (10-fold diluted with PBS) was added to each well, incubated at 37°C for 1 h, and washed three times with PBST. Added 100 µL of 0.1 µg/mL HRP-labelled goat anti-mouse IgG antibody, incubated at 37°C for 1 h, and washed three times with PBST. Added 100 µL TMB substrate and incubated at room temperature for 10 min. Added 50 µL of 2 M H₂SO₄ to terminate the reaction. The absorbance at a wavelength of 450 nm was measured and recorded as OD₂.

### 4) Detecting the binding strength of the antibody in the cell culture supernatant to the antigen by the indirect ELISA method

The G17 antigen (a conjugate of G17 and BSA) was diluted to 1 µg/mL with a pH 9.6 carbonate buffer, added to ELISA plates at 100 µl/well, incubated at 4°C for 12 h, washed three times with PBST. Added 200 µL of 1% OVA (ovalbumin), incubated at 37°C for 2 h, and washed once with PBST. 100 µL of the cell culture solution (10-fold diluted with PBS) was added to each well, incubated at 37°C for 1 h, and washed three times with PBST. Added 100 µL of 0.1 µg/mL HRP-labelled goat anti-mouse IgG antibody, incubated at 37°C for 1 h, and washed three times with PBST. Added 100 µL TMB substrate and incubated at room temperature for 10 min, and 50 µL of 2 M H₂SO₄ was added to terminate the reaction. The absorbance at a wavelength of 450 nm was measured and recorded as OD₃.

### 5) Screening and subcloning of cells and the large-scale preperation of the target antibody

The cultured cells were divided into two groups according to the single colony wells and the multiple colony wells. The first group was sorted according to OD₁/OD₂ from high to low, preferably the first ten strains of cells (named GS1-GS10 successively) were subject to an expansion culture.

The second group was sorted according to OD₁/OD₃ from high to low, and the first 20-well cells were subject to subcloning, respectively. The subcloned cells were detected according to the methods of 1) and 3), and ten cells with higher OD₁/OD₂ were selected (named GP1-GP10 successively) to be subject to an expansion culture.

A batch of Balb/c mice were injected intraperitoneally with 500 µL Freund's incomplete adjuvant 7 days in advance, and each mouse was injected with 0.5 × 10⁶ hybridoma cells of an expansion culture. Mice ascites was collected after seven days of feeding and the antibody was purified by n-caprylic acid - ammonium sulfate method.

### 6) The application effect of antibody pairs

Two detection systems were selected to evaluate the obtained antibody pairs.

ELISA platform: 1 µg/mL G-1 was used to coat ELISA plates, the newly selected antibody was labelled with HRP, and diluted with PBS to 0.1 µg/mL, and G17 antigen was detected by the sandwich method to evaluate the pairing effect.

Specifically, the previously screened antibody was labelled with HRP using the labelling method of the T2 antibody in Example 1. The antibody G-1 was diluted to 1 µg/mL with a pH 9.6 carbonate buffer. Added them to ELISA plates at 100 µl/well, incubated at 4°C for 12 h, washed three times with PBST. Added 200 µL of 1% OVA, incubated at 37°C for 2 h, and washed once with PBST. Added 50 µL of 0.1 ng/mL G17 antigen and 50 µL of 0.1 µg/mL HRP-labelled freshly prepared antibody to each well, incubated at 37°C for 1 h and washed three times with PBST. Added 100 µL TMB substrate and incubated at room temperature for 10 min. Added 50 µL of 2 M H₂SO₄ to terminate the reaction. The absorbance at a wavelength of 450 nm was measured.

CLIA platform: G-1 was used to coat the magnetic micro-beads at a mass ratio of 1 : 100, and the newly screened antibody was labelled with ABEI, and different concentration of G17 was detected by the sandwich method to evaluate the pairing effect.

Specifically, 2 mg of the screened antibody was brought to 200 µL binding buffer (0.1 mol/L 2-[N-morpholino]ethanesulfonic acid, pH 4.5), 2 mg ABEI was dissolved into 500 µL binding buffer, and the two solutions were mixed; 10 mg EDC (diethylamine carbide) was weighted and added to the super-pure water, and 100 µL of the solution was immediately added to the ABEI antibody mixture in the last step, and incubated at room temperature for 2 h. The product was dialyzed against 4 L PBS at 4°C for 8 h, up to the completion of the labelling. 1 mg of G-1 antibody and 100 mg of magnetic micro-beads were weighed in PBS, mixed and incubated at 40°C for 2 h to complete the coating process. Added 20 µg magnetic micro-beads coated with G-1 antibody to the reaction cup of Maglumi 2000 full-automatic biochemical luminescence instrument, then added 100 µL of 0.1 ng/mL G17 antigen and 100 µL ABEI labelled antibody for reaction at 37°C for 10 min. Cleaning solution was used to wash three times, 100 µL substrate A (NaOH) and substrate B (H₂O₂) were added and immediately sent to the measurement chamber to measure the relative luminous intensity Rlu.

### The pairing results of 20 antibodies obtained in Example 2 with G-1

Through the preliminary screening of F(ab)₂ and goat anti-mouse IgG Fc, we obtained 371 wells of cells with OD₄₅₀ > 1.5, wherein 63 wells of cells were single colonies (single cell clumps formed by one cell growth division) and the remaining 308 wells of cells were multiple colonies. 10 strains of cells S1-S10 were selected from the 63 single colony wells through sorting according to OD₁/OD₂; 20 strains of cells were selected from the 278 multiple colony wells through sorting according to OD₁/OD₃, and further selected 10 strains of cells P1-P10 by sorting according to OD₁/OD₂ after subcloning.

G-1 was diluted to 1 µg/mL using CBS (pH 9.6), 100 µL of which was taken to coat the ELISA plates, GS1-GS10 and GP1-GP10 were labelled with HRP, added 100 µL to each well at 0.1 µg/mL, and 100 pg/mL of G17 antigen was detected by the sandwich ELISA method. The results showed that all the antibodies were well paired with G-1, and all OD₄₅₀ were greater than 2.5 except for GS7, GS8, GS9 and GS10.

Table 3 shows the pairing of GS1-GS10, GP1-GP10 with G-1 evaluated by the sandwich ELISA method.

**Table 3**

| The concentration of antigen | GS1 | GS2 | GS3 | GS4 | GS5 |
|---|---|---|---|---|---|
| 100 pg/mL | 3.29 | 3.31 | 3.12 | 2.71 | 2.76 |

| | GS6 | GS7 | GS8 | GS9 | GS10 |
|---|---|---|---|---|---|
| 100 pg/mL | 2.56 | 2.47 | 2.25 | 2.08 | 2.14 |

| | GP1 | GP2 | GP3 | GP4 | GP5 |
|---|---|---|---|---|---|
| 100 pg/mL | 3.43 | 3.52 | 3.38 | 3.34 | 3.32 |

| | GP6 | GP7 | GP8 | GP9 | GP10 |
|---|---|---|---|---|---|
| 100 pg/mL | 3.25 | 3.02 | 2.96 | 2.83 | 2.91 |

| | | | | | |
|---|---|---|---|---|---|
| Note: The data in the table indicate OD₄₅₀, and the detection background value (under the same condition other than the absence of antigen) is less than 0.2. | | | | | |

G-1 was used to coat the magnetic micro-beads produced by Snibe company, GS1-GS10 and GP1-GP10 were labelled with ABEI, and 100 pg/mL G17 antigen was detected by the sandwich method using the Snibe Maglumi system. The results show that GS2, GP1 and GP8 were paired well with G1, among which GS2 and GP1 had the best effect.

Table 4 shows the pairing of GS1-GS10, GP1-GP10 with G-1 evaluated by the sandwich CLIA method.

**Table 4**

| The concentration of antigen | GS1 | GS2 | GS3 | GS4 | GS5 |
|---|---|---|---|---|---|
| 0 pg/mL | 809 | 2430 | 5240 | 30907 | 18973 |
| 100 pg/mL | 43802 | 1107631 | 23464 | 58573 | 19645 |

| | GS6 | GS7 | GS8 | GS9 | GS10 |
|---|---|---|---|---|---|
| 0 pg/mL | 3972 | 13451 | 3451 | 8512 | 5634 |
| 100 pg/mL | 8716 | 22142 | 5632 | 14421 | 6421 |

| | GP1 | GP2 | GP3 | GP4 | GP5 |
|---|---|---|---|---|---|
| 0 pg/mL | 2871 | 14512 | 16752 | 12133 | 13763 |
| 100 pg/mL | 1386542 | 56214 | 47532 | 245231 | 21765 |

| | GP6 | GP7 | GP8 | GP9 | GP10 |
|---|---|---|---|---|---|
| 0 pg/mL | 8965 | 5674 | 3747 | 16785 | 1652 |
| 100 pg/mL | 13341 | 8532 | 464673 | 18551 | 2043 |

| | | | | | |
|---|---|---|---|---|---|
| Note: The data in Table 4 indicate the relative luminescent intensity. | | | | | |

### Example 3

### The preparation of anti-folate binding protein antibody pair

Folic acid binding protein (FABP), also known as folic acid receptor protein, plays an important role in cell division, proliferation and sound field, and is generally detected by using the double antibody sandwich method. In the previous work we obtained an antibody F-1 with a relatively high affinity for FABP. In this example we would prepare the pairing antibody of F-1.

### 1) Acquisition of F(ab)₂

2 mL of 3 mg/mL purified F-1 was dialyzed against 200 mL of acetic acid buffer (pH 4.0) at 4°C for 4 h, the concentration was re-measured and adjusted to 2 mg/mL using acetate buffer with the same pH. The pepsin concentration of 0.1 mg/mL was formulated with the acetate buffer (pH 4.0), and 1 mL of the solution was added to the above described antibody solution to make the enzyme to antibody of 1 : 20 to react at 37°C for 4 h. The reaction was terminated by adding 100 µL of 2 mol/L Tris base. The mixture was transferred to a dialysis bag and dialyzed against 1 L PBS of pH 8.0 at 4°C. The dialysate was loaded onto a 5 mm × 100 mm protein A cross-linked agarose gel CL-4B chromatographic column and the unbound effluent is collected. The effluent was concentrated to 4 mL and loaded onto a 26 mm × 900 mm polyacrylamide dextran S-200 Superfine chromatographic column to collect the component with the molecular weight of 110 kDa. The above described product was identified using 10% SDS non-reducing polyacrylamide gel electrophoresis, showing a band only at 110 kDa, a single band at 25 kDa was shown using non-reducing polyacrylamide gel electrophoresis. A280 was detected to determine the final concentration of F(ab)₂.

### 2) Screening the antibodies that can be paired with F-1 by the sandwich ELISA method

The mature Balb/c mice immunized with FABP were challenged by intraperitoneal injection of 300 µg of FABP three days in advance. The spleen cells were harvested and ground to obtain lymphocyte, and the cell fusion of the lymphocyte and mice myeloma cell sp2/0 was facilitated by using PEG1500. Put them in 20 pieces of 96-well cell culture plates after fusion. Cultured at 37°C, 5% CO₂ for 7 days, replaced the fresh medium (80% RPMI1640, 20% fetal bovine serum) and cultured for another day.

The F-1 F(ab)₂ solution was diluted to 1 µg/mL with a pH 9.6 carbonate buffer and was added to 20 pieces of 96-well ELISA plates at 100 µl/well, incubated at 4°C for 12 h, and washed with PBST (containing 0.05% Tween-20 in PBS buffer, pH 7.4). Added 200 µLof 1% OVA (ovalbumin) to incubate at 37°C for 2 h to block the un-reacted site on the ELISA plate and washed once with PBST. 50 µL of 50 ng/mL FABP antigen and 50 µL of cell culture solution were added to each well, incubated at 37°C for 1 h and washed three times with PBST. Added 100 µL of 0.1 µg/mL HRP-labelled goat anti-mouse IgG Fc antibody, incubated at 37°C for 1 h, and washed three times with PBST. Added 100 µL TMB substrate and incubated at room temperature for 10 min. Added 50 µL of 2 M H₂SO₄ to terminate the reaction. The absorbance at a wavelength of 450 nm was measured and recorded as OD₁. Cell cultures with OD₁ > 1.5 were subjected to the detection of the next two steps.

### 3) Detecting the relative amount of total antibodies in the cell culture supernatant by the double antibody sandwich ELISA method

The mixture of goat anti-mouse IgG1, IgG2a, IgG2b, IgG3 antibodies (no cross-link from each other) was diluted to 2 µg/mL with a pH 9.6 carbonate buffer. Added them to ELISA plates at 100 µl/well, incubated at 4°C for 12 h, and washed with PBST (containing 0.05% Tween-20 in PBS buffer, pH 7.4). Added 200 µL of 1% OVA (ovalbumin), incubated at 37°C for 2 h, and washed once with PBST. 100 µL of the cell culture solution (10-fold diluted with PBS) was added to each well, incubated at 37°C for 1 h, and washed three times with PBST. Added 100 µL of 0.1 µg/mL HRP-labelled goat anti-mouse IgG antibody, incubated at 37°C for 1 h, and washed three times with PBST. Added 100 µL TMB substrate and incubated at room temperature for 10 min, and 50 µL of 2 M H₂SO₄ was added to terminate the reaction. The absorbance at a wavelength of 450 nm was measured and recorded as OD₂.

### 4) Detecting the binding strength of the antibody in the cell culture supernatant to the antigen by the indirect ELISA method

The FABP antigen was diluted to 1 µg/mL with a pH 9.6 carbonate buffer. Added them to ELISA plates at 100 µl/well, incubated at 4°C for 12 h, and washed three times with PBST. Added 200 µL of 1% OVA (ovalbumin), incubated at 37°C for 2 h, and washed once with PBST. 100 µL of the cell culture solution (10-fold diluted with PBS) was added to each well, incubated at 37°C for 1 h, and washed three times with PBST. Added 100 µL of 0.1 µg/mL HRP-labelled goat anti-mouse IgG antibody, incubated at 37°C for 1 h, and washed three times with PBST. Added 100 µL TMB substrate and incubated at room temperature for 10 min. Added 50 µL of 2 M H₂SO₄ to terminate the reaction. The absorbance at a wavelength of 450 nm was measured and recorded as OD₃.

### 5) Screening and subcloning of cells and the large-scale preperation of the target antibody

The cultured cells were divided into two groups according to the single colony wells and the multiple colony wells. The first group was sorted according to OD₁/OD₂ from high to low, preferably the first ten cell strains (named FS1-FS10 successively) were subject to an expansion culture.

The second group was sorted according to OD₁/OD₃ from high to low, and the first 20-well cells were subject to subcloning, respectively. The subcloned cells were detected according to the methods of 1) and 3), and ten cells with higher OD₁/OD₂ were are selected (named FP1-FP10 successively) to be subject to an expansion culture.

A batch of Balb/c mice were injected intraperitoneally with 500 µL Freund's incomplete adjuvant 7 days in advance, and each mouse was injected with 0.5 × 10⁶ hybridoma cells of expansion culture. Mice ascites was collected after seven days of feeding and the antibody was purified by n-caprylic acid - ammonium sulfate method.

### 6) The application effect of antibody pairs

We selected two detection systems to evaluate the obtained antibody pairs.

ELISA platform: 1 µg/mL F-1 was used to coat ELISA plates, the newly selected antibody was labelled with HRP, and diluted with PBS to 0.1 µg/mL , and FABP antigen was detected by the sandwich method to evaluate the pairing effect.

Specifically, the previously screened antibody was labelled with HRP using the labelling method of the T2 antibody in Example 1. The antibody F-1 was diluted to 1 µg/mL with a pH 9.6 carbonate buffer. Added them to ELISA plates at 100 µl/well, incubated at 4°C for 12 h, and washed three times with PBST. Added 200 µL of 1% OVA, incubated at 37°C for 2 h, and washed once with PBST. Added 50 µL of 50 ng/ml FABP and 50 µL of 0.1 µg/mL HRP-labelled freshly prepared antibody to each well, incubated at 37°C for 1 h and washed three times with PBST. Added 100 µL TMB substrate and incubated at room temperature for 10 min. Added 50 µL of 2 M H₂SO₄ to terminate the reaction. The absorbance at a wavelength of 450 nm was measured.

CLIA platform: F-1 was used to coat the magnetic micro-beads at a mass ratio of 1 : 100, and the newly FS1-FS10 and FP1-FP10 antibody was labelled with ABEI, and FABP antigen was detected by the sandwich method to evaluate the pairing effect.

Specifically, 2 mg of the screened antibody was brought to 200 µL binding buffer (0.1 mol/L 2-[N-morpholino]ethanesulfonic acid, pH 4.5), 2 mg ABEI was dissolved into 500 µL binding buffer, and the two solutions were mixed; 10 mg EDC (diethylamine carbide) was weighted and added to the super-pure water, and 100 µLof the solution was immediately added to the ABEI antibody mixture obtained in the last step, and incubated at room temperature for 2 h. The product was dialyzed against 4 L PBS at 4°C for 8 h, up to the completion of the labelling. 1 mg of F-1 antibody and 100 mg of magnetic micro-beads produced by Snibe company were weighed and added to PBS to be mixed, and incubated at 40°C for 2 h to complete the coating process. The magnetic micro-beads coated with F-1 antibody were loaded to the reaction cup using Maglumi full-automatic biochemical luminescence instrument, and then added 100 µL of 50 ng/ml FABP and 100 µL ABEI labelled antibody for reaction at 37°C for 10 min. Cleaning solution was used to wash three times, 100 µL substrate A (NaOH) and substrate B (H₂O₂) were added and immediately sent to the measurement chamber to measure the relative luminous intensity Rlu.

### The pairing results of the 20 antibodies obtained in Example 3 with F-1

Through the preliminary screening of F(ab)₂ and goat anti-mouse IgG Fc, we obtained 480 wells of cells with OD₄₅₀ > 1.5, wherein 72 wells of cells were single colonies (single cell clumps formed by one cell growth division) and the remaining 408 wells of cells were multiple colonies. 10 cell strains FS1-FS10 were selected from the 72 single colony wells through sorting according to OD₁/OD₂; 20 strains of cells were selected from 408 multiple colony wells through sorting according to OD₁/OD₃, and 10 strains of cells FP1-FP10 were further selected by sorting according to OD₁/OD₂ after subcloning.

F-1 was diluted to 1 µg/mL using CBS (pH 9.6), 100 µL of which was taken to coat the ELISA plates, FS1-FS10 and FP1-FP10 were labelled with HRP and diluted to 0.1 µg/mL using PBS, 100 µL of which was added to each well, and 50 ng/mL FABP was detected by the sandwich ELISA method. The results showed that all the antibodies were well paired with F-1, and all OD₄₅₀ were greater than 2.5 except for GS7, GS8, GS9 and GS10.

Table 5 shows the pairing of FS1-FS10 and FP1-FP10 with F-1 evaluated by the sandwich ELISA method.

**Table 5**

| The concentration of antigen | FS1 | FS2 | FS3 | FS4 | FS5 |
|---|---|---|---|---|---|
| 50 ng/ml | 3.82 | 3.56 | 3.11 | 2.65 | 2.78 |

| | FS6 | FS7 | FS8 | FS9 | FS10 |
|---|---|---|---|---|---|
| 50 ng/ml | 2.68 | 2.52 | 2.32 | 2.41 | 2.14 |

| | FP1 | FP2 | FP3 | FP4 | FP5 |
|---|---|---|---|---|---|
| 50 ng/ml | 3.75 | 3.82 | 3.85 | 3.46 | 3.65 |

| | FP6 | FP7 | FP8 | FP9 | FP10 |
|---|---|---|---|---|---|
| 50 ng/ml | 3.55 | 3.46 | 2.98 | 3.07 | 3.11 |

| | | | | | |
|---|---|---|---|---|---|
| Note: The data in the table indicate OD₄₅₀, and the detection background value (under the same condition other than the absence of antigen) is less than 0.2. | | | | | |

F-1 was used to coat the magnetic micro-beads produced by Snibe company, FS1-FS10 and FP1-FP10 were labelled with ABEI, and 50 ng/ml FABP antigen was detected by the sandwich method in the Maglumi system. The results showed that FS-3, FP-1, FP-2, FP-5, and FP-7 were paired well with F-1, among which FS-3, FP-1 and FP-7 had the best effect.

Table 6 shows the pairing of FS1-FS10 and FP1-FP10 with F-1 evaluated on the CLIA platform.

**Table 6**

| The concentration of antigen | FS1 | FS2 | FS3 | FS4 | FS5 |
|---|---|---|---|---|---|
| 0 pg/ml | 5896 | 12474 | 8672 | 35278 | 6788 |
| 50 ng/ml | 173110 | 214054 | 1836981 | 82578 | 124274 |

| | FS6 | FS7 | FS8 | FS9 | FS10 |
|---|---|---|---|---|---|
| 0 pg/ml | 8677 | 12571 | 8674 | 6587 | 5874 |
| 50 ng/ml | 65814 | 24157 | 10254 | 6438 | 9375 |

| | FP1 | FP2 | FP3 | FP4 | FP5 |
|---|---|---|---|---|---|
| 0 pg/ml | 5387 | 28797 | 16975 | 6837 | 7854 |
| 50 ng/ml | 1987752 | 988725 | 268774 | 367784 | 1355789 |

| | FP6 | FP7 | FP8 | FP9 | FP10 |
|---|---|---|---|---|---|
| 0 pg/ml | 24875 | 8074 | 8982 | 12745 | 4387 |
| 50 ng/ml | 687724 | 1386489 | 35047 | 254774 | 82449 |

| | | | | | |
|---|---|---|---|---|---|
| Note: The data in Table 4 indicate the relative luminescent intensity. | | | | | |

### Comparative Example 4

### FABP antibody pairs were prepared using the commonly used method

The commonly used strategies are as the method of preparing antibody pairs described in the reference "The preparation of anti-human alpha-fetoprotein monoclonal antibody and the establishment of double antibody sandwich ELISA detection technology, Sun Yifan, Chinese Medicinal Biotechnology, August 2014, Vol. 9, No. 4".

### 1) Acquisition of hybridoma cells

The mature Balb/c mice immunized with FABP were challenged by intraperitoneal injection of 300 µg of FABP three days in advance. The spleen cells were harvested and ground to obtain lymphocyte, and the cell fusion of the lymphocyte and mice myeloma cell sp2/0 was facilitated by using PEG1500. Put them in 10 pieces of 96-well cell culture plates after fusion. Cultured at 37°C, 5% CO₂ for 7 days, replaced the fresh medium (80% RPMI1640, 20% fetal bovine serum) and cultured for another day.

### 2) Screening the antibodies that bind to FABP antigens

The FABP was diluted to 1 µg/mL using a pH 9.6 carbonate buffer and was added to the ELISA plates at 100 µl/well, incubated at 4°C for 12 h, and washed three times with PBST (containing 0.05% Tween-20 in PBS buffer, pH 7.4). Added 200 µL of 1% OVA (ovalbumin), incubated at 37°C for 2 h, and washed once with PBST. 100 µL of the above described cell culture solution (10-fold diluted with PBS) was added to each well, incubated at 37°C for 1 h, and washed three times with PBST. Added 100 µL of 0.1 µg/mL HRP-labelled goat anti-mouse IgG antibody, incubated at 37°C for 1 h, and washed three times with PBST. Added 100 µL TMB substrate and incubated at room temperature for 10 min. Added 50 µL of 2 M H₂SO₄ to terminate the reaction. The absorbance at a wavelength of 450 nm was measured. 20-well cells were selected according to the absorbance from high to low.

### 3) Screening paired antibodies

Twenty strains of cells were subcloned, and the individual cell strains were isolated and subject to an expansion culture. A large amount of antibodies secreted by each cell strain were obtained as that in Example 1, and the 20 antibodies obtained were named FE1-FE20 successively.

A total of 21 antibodies of FE1-FE20 and F-1 were combined in pairs (also can be self-combined), forming 441 (21 × 21) combinations. In an antibody pair of these combinations, one was used to coat the ELISA plate and another one was labelled with HRP. The FABP antigens were detected by the sandwich ELISA method. The cells with positive results were detected (OD450 > negative control wells ± double variance, negative control wells are all the same except of absence of antigen). The procedure of labelling and detection is the same as that in Example 3.

### 4) The application effect of antibody pairs

Using the CLIA sandwich method, the pairing successful antibody pairs were tested for further pairing against the Maglumi system of Snibe company. Specifically, using the method in Example 3, one of an antibody pair was used to coat the magnetic micro-beads produced by Snibe and another one was labelled with ABEI. The FABP was detected using the Maglumi 2000 sandwich method to make evaluation.

### The pairing results of the 20 antibodies obtained in the comparative example

1321 wells of cells were screened by the indirect method and detected to be positive (OD₄₅₀ > 1.5), and among which 20 strains with relatively high binding value were selected for paired detection. A total of 21 antibodies of FE1-FE20 and F-1 were pair-matched into 441 groups. One antibody of each group was labelled with HRP and another one was used to coat the ELISA plate. 50 ng/ml FABP was detected by the sandwich ELISA method. The pairing results showed that there were 62 combinations with OD₄₅₀ > 1.5, of which only 6 combinations had OD₄₅₀ > 2.5: FE2/FE12, FE2/FE17, FE5/FE13, FE7/FE14, FE8/FE18 and FE10/F-1.

Table 7 shows the screening of anti-FABP antibody pairs using the conventional method.

**Table 7**

| | FE12 | FE13 | FE14 | FE17 | FE18 | F-1 |
|---|---|---|---|---|---|---|
| FE2 | 2.84 | 0.21 | 0.13 | 3.01 | 0.74 | 0.67 |
| FE5 | 0.24 | 2.69 | 0.29 | 0.18 | 0.67 | 1.67 |
| FE7 | 0.28 | 0.67 | 2.98 | 0.21 | 0.32 | 0.43 |
| FE8 | 0.11 | 0.18 | 0.26 | 0.19 | 2.56 | 0.58 |
| FE10 | 0.28 | 0.07 | 0.18 | 0.24 | 0.47 | 3.12 |

Each of the above six antibody pairs were labelled with ABEI and used to coat magnetic beads, respectively, and 50 ng/mL FABP antigen was detected by the sandwich method on Maglumi 2000. The results showed that only FE2/FE17 was paired to some extent, and the other antibody pairs were not suitable for Maglumi system.

Table 8 shows the evaluation of the pairing of six antibody pairs by the CLIA sandwich method.

**Table 8**

| | FE2/FE12 | FE2/FE17 | FE5/FE13 | FE7/FE14 | FE8/FE18 | FE10/F-1 |
|---|---|---|---|---|---|---|
| 0 pg/ml | 12841 | 8941 | 6874 | 5681 | 6624 | 12571 |
| 50 ng/ml | 26574 | 541001 | 12574 | 9824 | 8974 | 35714 |

### Result analysis

As can be seen from Table 1 in Example 1, antibody T-1 could continue to be paired with T-2 for sandwich detection of TPO antigen after it was digested into Fab' fragment and F(ab)₂ by enzyme, wherein the pairing effect of F(ab)₂ was close to that of the intact antibody. In Table 2, the T-2 antibody was present in the cell culture supernatant. The HRP-labelled goat anti-mouse IgG Fc was used as the second antibody. If the intact T-1 was used as the capture antibody, since T-1 and T-2 have the same Fc fragment, the second antibody reacted with both of them. The detection value did not reduce with the decrease of the addition of the antigen, and thus cannot reflect the true pairing. However, if the Fab' fragment and F(ab)₂ were used as the capture antibody, the detection values were relatively high and reduced with the decrease of the addition of the antigen, indicating that both the Fab' fragment and F(ab)₂ of T-1 were paired well with the T-2 antibody in the cell culture supernatant and could be well detected. As the stability of F(ab)₂ was better than that of the monovalent Fab', it reflects the better pairing effect. To sum up, using the Fab' fragment and F(ab)₂ of an antibody as the capture antibody, it is possible to directly screen for antibody from the cell culture supernatant with which it is paired.

From Examples 2 and 3, it was found that all of the screened antibody could be used in the sandwich ELISA method, and the pairing strength was roughly in accordance with the sorting order during the screening, indicating that the optimized strategy was reasonable. From Tables 2 and 4, it was found that the resulting antibodies after two rounds of screening (GP1-GP10 and FP1-FP10) were superior to those of single-round acreening (GS1-GS10 and FS1-FS10), indicating that the pairing success rate was improved to some extent through two-round-screening of OD₁/OD₃ and OD₁/OD₂ compared with that of OD₁/OD₂ single-round-screening.

Screening was performed using all the cell colonies (5,000-10,000) of one cell fusion by the sandwich method in Example 3 and 408 wells of cells were obtained. All the 20 strains of cells through screening can be paired with the existing high-sensitivity antibody F-1 to be used in the sandwich ELISA method, among which five strains can be paired with F-1 to be used in the sandwich CLIA method. The comparative Example firstly screened 20 antibodies that can bind to the antigen, and then these antibodies were prepared in large-scale, purified and labelled with HRP. Only six antibody pairs that can be used in the sandwich ELISA method were screened from the 441 combinations by the chessboard method, among which one antibody pair can be used in the CLIA sandwich method.

The method that antibody coats the ELISA plate in ELISA is the physical adsorption, the used marker HRP is macromolecular protein, and generally takes a 1 : 1 molar ratio when it labels antibody; however, the process that antibody coats magnetic beads in CLIA and ratio that the antibody is labelled with small molecule compounds ABEI are all of relatively large difference from that in ELISA, and so the impact on the nature of the antibody itself is not the same. In addition, CLIA amplification signal function far exceeds the ELISA, and the requirements on the sensitivity of the antibody are higher. Therefore, only a small fraction of the antibody pairs that can be applied to ELISA are suitable for CLIA.

Comparing Example 3 and the comparative Example, we found that the former only required the use of antibody in the cell culture supernatant when performing the pairing screening, while the latter required the prepared antibodies; the former did not need the purification and labelling of antibody in the screening process, while the latter must use the antibodies purified and labelled with HRP; similarly for the 20 obtained antibodies, in the former all could be paired with the existing high-sensitivity antibodies, while the latter obtained only six antibody pairs, of which only one could be paired with the existing high-sensitivity antibodies; the former obtained five antibody pairs that can be used in the CLIA sandwich method, while the latter obtained only one pair with the pairing effect not as good as the former.

From the above description, it can be seen that the above-described examples of the present application achieved the following technical effects:
1) The pairing detection can be directly performed with the cell culture supernatant, which greatly expands the source of antibody to be detected and does not need labelling and purification in the screening process;
2) When too many antibodies are obtained in the preliminary screening, effective evaluation system is established for futher screening, which saves manpower while greatly increasing the success rate.

## Claims

1. A method for preparing an antibody pair, comprising: using an antigen-binding fragment of an existing antibody as a capture antibody and using an anti-crystallizable fragment antibody as a labelled antibody to directly screen a target antibody which can be paired with said existing antibody from cell culture supernatant; the antigen-binding fragment of said existing antibody is Fab' or F(ab)₂, and said labelled antibody is an anti-Fc antibody.

2. The method according to claim 1, wherein the antigen-binding fragment of said existing antibody is F(ab')₂; further comprising: optimizing the screened antibodies according to the total antibody content in said cell culture supernatant or the binding strength of an antibody to be screened in said cell culture supernatant to a target antigen, and said cell culture supernatant is from hybridoma cells.

3. The method according to claim 1, wherein said labelled antibody is labelled with a tracer marker, which directly or indirectly labels said labelled antibody.

4. The method according to claim 3, wherein said tracer marker is selected from at least one of an enzyme label, a fluorescent dye, a chemiluminescent dye, isoluminol and its derivatives and a radioactive label.

5. The method according to claim 2, wherein said optimizing step comprises:
a) detecting the screened antibodies that can be paired with the capture antibody in the cell culture supernatant by the sandwich method, and recording the resulting signal as OD₁;
b) detecting the total antibody content in said cell culture supernatant, and recording the resulting signal as OD₂;
c) detecting the binding strength of the antibody to be screened in said cell culture supernatant to the target antigen, and recording the resulting signal as OD₃; and
d) evaluating and optimizing the antibody screened in said step a) based on the OD₁/OD₂ or OD₁/OD₃ ratio.

6. The method according to claim 5, wherein said step d) comprises:
1) if the cell culture is a single cell strain cell culture, then the antibody with higher OD₁/OD₂ is paired better with said existing antibody;
2) if the cell culture is multiple cell strains cell culture, then the cell culture with higher OD₁/OD₃ is selected to be separated into single cells for continuous culture so as to obtain new cell cultures, and the new cell cultures are further optimized following said step 1).

7. The method according to claim 6, wherein said step d) specifically comprises:
dividing said cell cultures into two groups according to single colony wells and multiple colony wells;
sorting the group of the single colony wells according to OD₁/OD₂ from high to low, and selecting the first N cell strains, wherein said N is 1-20;
sorting the group of the multiple colony wells according to OD₁/OD₃ from high to low, and selecting the first M cell wells for subcloning, respectively, and then detecting the subcloned cells according to the methods of said step a) and said step b) and selecting N cell strains with higher OD₁/OD₂, wherein said M is 1-40.

8. The method according to claim 5, wherein said step b) specifically comprises: detecting the total antibody content in the cell culture supernatant using an antibody that specifically binds to an antibody conserved region or an immunoglobulin binding protein.

9. The method according to claim 8, wherein said antibody that specifically binds to an antibody conserved region is selected from the mixture of two or more strains of goat anti-mouse IgM, goat anti-mouse IgA, goat anti-mouse IgD, goat anti-mouse IgG1, goat anti-mouse IgG2a, goat anti-mouse IgG2b, goat anti-mouse IgG3, rabbit anti-mouse IgM, rabbit anti-mouse IgA, rabbit anti-mouse IgD, rabbit anti-mouse IgG1, rabbit anti-mouse IgG2a, rabbit anti-mouse IgG2b and rabbit anti-mouse IgG3, and said immunoglobulin binding protein is a Staphylococcus protein A and/or a Streptococcus protein G.

10. The method according to claim 5, wherein said step c) specifically comprises: coating the target antigen on a solid phase, and detecting the binding strength of the antibody to be screened in said cell culture supernatant to the target antigen by means of a second antibody.

11. The method according to claim 6, wherein said existing antibody is any one of the group consisting of an anti-gastrin-17 antibody, an anti-folate binding protein antibody, an anti-thyroid peroxidase antibody, an anti-thyroglobulin antibody, an anti-insulin antibody, an anti-ferritin antibody, an anti-alpha-fetoprotein antibody, an anti-carcinoembryonic antibody, an anti-prostate specific antigen antibody, an anti-luteinizing hormone antibody, an anti-prolactin antibody, an anti-human chorionic gonadotrnpin antibody, an anti-neuron-specific enolase antibody, an anti-carbohydrate antigen 125 antibody , an anti-carbohydrate antigen 153 antibody, an anti-carbohydrate antigen 199 antibody, an anti-cytokeratin nineteen fragment antibody, an anti-carbohydrate antigen 724 antibody, an anti-carbohydrate antigen 242 antibody, an anti-growth hormone antibody, an anti-myoglobin antibody, an anti-carbohydrate antigen 50 antibody, an anti-C reactive Protein antibody, an anti-corticotropin antibody, an anti-creatine kinase isoenzyme antibody, an anti-Sangtec-100 protein antibody, an anti-laminin antibody, an anti- type IV collagen antibody, an anti-brain natural peptide N-terminal precursor protein antibody, an anti-troponin antibody, an anti-parathyroid hormone antibody, an anti-calcitonin antibody, an anti-procalcitonin antibody, an anti-prostate acid phosphatase antibody, an anti-osteocalcin antibody, an anti-pregnancy-associated protein A antibody, an anti-pepsinogen I antibody, an anti-pepsinogen II antibody, an anti-insulin-like growth factor antibody or an anti-D-dimer antibody.

12. The method according to any one of claims 1 to 11, further comprising the expansion of the cell culture containing the screened antibody and the large-scale preparation and purification of the target antibody.

13. Use of a kit in the preparation of an antibody pair, wherein the kit comprises:
a capture antibody, which is an antigen-binding fragment of an existing antibody immobilized on a solid phase medium, the antigen-binding fragment of said existing antibody is Fab' or F(ab)₂; and
a labelled antibody, which is an anti-crystallizable fragment antibody; and
a target antigen which directly or indirectly binds to a solid phase medium; and
an antibody that specifically binds to an antibody conserved region or an immunoglobulin binding protein.

14. The use according to claim 13, wherein the antigen-binding fragment of said existing antibody is F(ab')₂.

15. The use according to claim 13, wherein said labelled antibody is labelled with a tracer marker, which directly or indirectly labels said labelled antibody.

16. The use according to claim 13, wherein said tracer marker is selected from at least one of, an enzyme label, a fluorescent dye, a chemiluminescent dye, isoluminol and its derivatives and a radioactive label.

17. The use according to claim 13, wherein said antibody that specifically binds to an antibody conserved region is selected from the mixture of two or more strains of goat anti-mouse IgM, goat anti-mouse IgA, goat anti-mouse IgD, goat anti-mouse IgG1, goat anti-mouse IgG2a, goat anti-mouse IgG2b, goat anti-mouse IgG3, rabbit anti-mouse IgM, rabbit anti-mouse IgA, rabbit anti-mouse IgD, rabbit anti-mouse IgG1, rabbit anti-mouse IgG2a, rabbit anti-mouse IgG2b and rabbit anti-mouse IgG3, and said immunoglobulin binding protein is a Staphylococcus protein A and/or a Streptococcus G protein.

18. The use according to claim 13, wherein said solid phase is an ELISA plate, magnetic beads or colloidal gold.

19. The use according to claim 13, wherein said existing antibody is any one of the group consisting of an anti-gastrin-17 antibody, an antifolate binding protein antibody, an anti-thyroid peroxidase antibody, an anti-thyroglobulin antibody, an anti-insulin antibody, an anti-ferritin antibody, an anti-alpha-fetoprotein antibody, an anti-carcinoembryonic antibody, an anti-prostate specific antigen antibody, an anti-luteinizing hormone antibody, an anti-prolactin antibody, an anti-human chorionic gonadotrnpin antibody, an anti-neuron-specific enolase antibody, an anti-carbohydrate antigen 125 antibody , an anti-carbohydrate antigen 153 antibody, an anti-carbohydrate antigen 199 antibody, an anti-cytokeratin nineteen fragment antibody, an anti-carbohydrate antigen 724 antibody, an anti-carbohydrate antigen 242 antibody, an anti-growth hormone antibody, an anti-myoglobin antibody, an anti-carbohydrate antigen 50 antibody, an anti-C reactive Protein antibody, an anti-corticotropin antibody, an anti-creatine kinase isoenzyme antibody, an anti-Sangtec-100 protein antibody, an anti-laminin antibody, an anti-type IV collagen antibody, an anti-brain natural peptide N-terminal precursor protein antibody, an anti-troponin antibody, an anti-parathyroid hormone antibody, an anti-calcitonin antibody, an anti-procalcitonin antibody, an anti-prostate acid phosphatase antibody, an anti-osteocalcin antibody, an anti-pregnancy-associated protein A antibody, an anti-pepsinogen I antibody, an anti-pepsinogen II antibody, an anti-insulin-like growth factor antibody or an anti-D-dimer antibody.

20. The use according to claim 13, wherein said kit further comprising: the reagents used in the expansion culture of cell cultures and the large-scale preparation and purification of the target antibody.

21. The use according to claim 13, wherein said kit is used in a semi-automatic or full-automatic immunoassay analyzer.

## Patentansprüche

1. Verfahren zur Herstellung eines Antikörperpaars, umfassend: Verwenden eines Antigen-bindenden Fragments eines bestehenden Antikörpers als Einfangantikörper und Verwenden eines antikristallisierbaren Fragmentantikörpers als markierten Antikörper, um einen Zielantikörper, der mit dem bestehenden Antikörper gepaart werden kann, aus dem Zellkulturüberstand direkt zu screenen; das Antigen-bindende Fragment des bestehenden Antikörpers ist Fab` oder F(ab)₂ und der markierte Antikörper ist ein Anti-Fc-Antikörper.

2. Verfahren nach Anspruch 1, wobei das Antigen-bindende Fragment des bestehenden Antikörpers F(ab')₂ ist;
zudem umfassend: Optimieren der gescreenten Antikörper gemäß dem Gesamtantikörpergehalt in dem Zellkulturüberstand oder der Bindungsstärke eines Antikörpers, der in dem Zellkulturüberstand an ein Zielantigen gescreent werden soll, und der Zellkulturüberstand stammt von Hybridomzellen.

3. Verfahren nach Anspruch 1, wobei der markierte Antikörper mit einem Tracermarker markiert ist, der den markierten Antikörper direkt oder indirekt markiert.

4. Verfahren nach Anspruch 3, wobei der Tracermarker aus mindestens einer Enzymmarkierung, einem Fluoreszenzfarbstoff, einem Chemilumineszenzfarbstoff, Isoluminol und seinen Derivaten und einer radioaktiven Markierung ausgewählt ist.

5. Verfahren nach Anspruch 2, wobei der Optimierungsschritt umfasst:
a) Erkennen der gescreenten Antikörper, die mit dem Einfangantikörper im Zellkulturüberstand gepaart werden können, durch das Sandwich-Verfahren und Aufzeichnen des resultierenden Signals als OD₁;
b) Erkennen des Gesamtantikörpergehalts im Zellkulturüberstand und Aufzeichnen des resultierenden Signals als OD₂;
c) Erkennen der Bindungsstärke des zu screenenden Antikörpers in dem Zellkulturüberstand zum Zielantigen und Aufzeichnen des resultierenden Signals als OD₃; und
d) Bewerten und Optimieren des im Schritt a) gescreenten Antikörpers basierend auf dem OD₁/OD₂- oder OD_{1/}OD₃-Verhältnis.

6. Verfahren nach Anspruch 5, wobei der Schritt d) umfasst:
1) wenn die Zellkultur eine Einzelzell-Stammzellkultur ist, dann ist der Antikörper mit höherem OD₁/OD₂ mit dem bestehenden Antikörper besser gepaart;
2) wenn die Zellkultur eine Mehrzell-Stammzellkultur ist, dann wird die Zellkultur mit höherem OD₁/OD₃ ausgewählt, um für eine kontinuierliche Kultur in einzelne Zellen getrennt zu werden, um neue Zellkulturen zu erhalten, und die neuen Zellkulturen werden im Anschluss an Schritt 1) weiter optimiert.

7. Verfahren nach Anspruch 6, wobei der Schritt d) spezifisch umfasst:
Aufteilen der Zellkulturen in zwei Gruppen nach einzelnen Koloniemulden und mehrfachen Koloniemulden;
Sortieren der Gruppe der einzelnen Koloniemulden nach OD₁/OD₂ von hoch nach unten und Auswählen der ersten N-Zellstämme, wobei N 1-20 ist, Sortieren der Gruppe der mehrfachen Koloniemulden nach OD₁/OD₃ von hoch nach unten und Auswählen der ersten M-Zellmulden zum Subklonieren bzw. dann Erkennen der subklonierten Zellen nach den Verfahren von Schritt a) und Schritt b) und Auswählen von N-Zellstämmen mit höherem OD_{1/}OD₂, wobei M 1-40 ist.

8. Verfahren nach Anspruch 5, wobei der Schritt b) spezifisch umfasst: Erkennen des Gesamtantikörpergehalts im Zellkulturüberstand unter Verwendung eines Antikörpers, der spezifisch an eine Antikörper-konservierte Region oder ein Immunglobulin-bindendes Protein bindet.

9. Verfahren nach Anspruch 8, wobei der Antikörper, der spezifisch an eine Antikörper-konservierte Region bindet, aus der Mischung von zwei oder mehreren Stämmen von Ziegen-Anti-Maus-IgM, Ziegen-Anti-Maus-IgA, Ziegen-Anti-Maus-IgD, Ziegen-Anti-Maus-IgG1, Ziegen-Anti-Maus-IgG2a, Ziegen-Anti-Maus-IgG2b, Ziegen-Anti-Maus-IgG3, Kaninchen-Anti-Maus-IgM, Kaninchen-Anti-Maus-IgA, Kaninchen-Anti-Maus-IgD, Kaninchen-Anti-Maus-IgG1, Kaninchen-Anti-Maus-IgG2a, Kaninchen-Anti-Maus-IgG2b und Kaninchen-Anti-Maus-IgG3 ausgewählt ist und das Immunglobulinbindende Protein ist ein Staphylococcus-Protein A und/oder ein Streptococcus-Protein G.

10. Verfahren nach Anspruch 5, wobei der Schritt c) spezifisch umfasst: Beschichten des Zielantigens auf eine feste Phase und Erkennen der Bindungsstärke des zu screenenden Antikörpers in dem Zellkulturüberstand an das Zielantigen mittels eines zweiten Antikörpers.

11. Verfahren nach Anspruch 6, wobei der bestehende Antikörper einer aus der Gruppe ist, die aus einem Anti-Gastrin-17-Antikörper, einem Anti-Folatbindenden Protein-Antikörper, einem Anti-Schilddrüsen-Peroxidase-Antikörper, einem Anti-Thyreoglobulin-Antikörper, einem Anti-Insulin-Antikörper, einem Anti-Ferritin-Antikörper, einem Anti-Alpha-Fetoprotein-Antikörper, einem Anti-Karzinoembryonalen Antikörper, einem Anti-Prostataspezifischen Antigen-Antikörper, einem antiluteinisierenden Hormon-Antikörper, einem Anti-Prolaktin-Antikörper, einem Anti-Human-Choriongonadotropin-Antikörper, einem Anti-Neuronspezifischen Enolase-Antikörper, einem Anti-Kohlenhydrat-Antigen-125-Antikörper, einem Antikohlenhydrat-Antigen-153-Antikörper, einem Anti-Kohlenhydrat-Antigen-199-Antikörper, einem Anti-Cytokeratin-Neunzehn-Fragment-Antikörper, einem Anti-Kohlenhydrat-Antigen-724-Antikörper, einem Anti -Kohlenhydrat-Antigen-242-Antikörper, einem Anti-Wachstumshormon-Antikörper, einem Anti-Myoglobin-Antikörper, einem Anti-Kohlenhydrat-Antigen-50-Antikörper, einem Anti-C-reaktiven Protein-Antikörper, einem Anti-Corticotropin-Antikörper, einem Anti-Kreatinkinase-Isoenzym-Antikörper, einem Anti-Sangtec-100-Protein-Antikörper, einem Anti-Laminin-Antikörper, einem Anti-Typ-IV-Kollagen-Antikörper, einem Antigehirn-natürlichen Peptid-N-terminalen Vorläufer-Protein-Antikörper, einem Anti-Troponin-Antikörper, einem Anti-Parathyroid-Hormon-Antikörper, einem Anti-Calcitonin-Antikörper, einem Anti-Procalcitonin-Antikörper, einem Anti-Prostata-Säure-Phosphatase-Antikörper, einem Anti-Osteocalcin-Antikörper, einem Anti-Schwangerschaftassoziierten Protein-A-Antikörper, einem Anti-Pepsinogen-I-Antikörper, einem Anti-Pepsinogen-II-Antikörper, einem Antiinsulin-ähnlichen Wachstumsfaktor-Antikörper oder einem Anti-D-Dimer-Antikörper ausgewählt ist.

12. Verfahren nach einem der Ansprüche 1 bis 11, zudem umfassend die Expansion der Zellkultur, die den gescreenten Antikörper enthält, und die Herstellung und Reinigung des Zielantikörpers in großem Maßstab.

13. Verwendung eines Kits bei der Herstellung eines Antikörperpaars, wobei das Kit umfasst:
einen Einfangantikörper, der ein Antigen-bindendes Fragment eines bestehenden Antikörpers ist, der auf einem Festphasenmedium immobilisiert ist, wobei das Antigen-bindende Fragment des bestehenden Antikörpers Fab' oder F(ab)₂ ist; und
einen markierten Antikörper, der ein antikristallisierbarer Fragmentantikörper ist; und
ein Zielantigen, das direkt oder indirekt an ein Festphasenmedium bindet; und
einen Antikörper, der spezifisch an eine Antikörper-konservierte Region oder ein Immunglobulin-bindendes Protein bindet.

14. Verwendung nach Anspruch 13, wobei das Antigen-bindende Fragment des bestehenden Antikörpers F(ab')₂ ist.

15. Verwendung nach Anspruch 13, wobei der markierte Antikörper mit einem Tracermarker markiert ist, der den markierten Antikörper direkt oder indirekt markiert.

16. Verwendung nach Anspruch 13, wobei der Tracermarker aus mindestens einer Enzymmarkierung, einem Fluoreszenzfarbstoff, einem Chemilumineszenzfarbstoff, Isoluminol und seinen Derivaten und einer radioaktiven Markierung ausgewählt ist.

17. Verwendung nach Anspruch 13, wobei der Antikörper, der spezifisch an eine Antikörper-konservierte Region bindet, aus der Mischung von zwei oder mehreren Stämmen von Ziegen-Anti-Maus-IgM, Ziegen-Anti-Maus-IgA, Ziegen-Anti-Maus-IgD, Ziegen-Anti-Maus-IgG1, Ziegen-Anti-Maus-IgG2a, Ziegen-Anti-Maus-IgG2b, Ziegen-Anti-Maus-IgG3, Kaninchen-Anti-Maus-IgM, Kaninchen-Anti-Maus-IgA, Kaninchen-Anti-Maus-IgD, Kaninchen-Anti-Maus-IgG1, Kaninchen-Anti-Maus-IgG2a, Kaninchen-Anti-Maus-IgG2b und Kaninchen-Anti-Maus-IgG3 ausgewählt ist und das Immunglobulinbindende Protein ist ein Staphylococcus-Protein A und/oder ein Streptococcus-Protein G.

18. Verwendung nach Anspruch 13, wobei die feste Phase eine ELISA-Platte, Magnetkügelchen oder kolloidales Gold ist.

19. Verwendung nach Anspruch 13, wobei der bestehende Antikörper einer aus der Gruppen ist, die aus einem Anti-Gastrin-17-Antikörper, einem Anti-Folat-bindenden Protein-Antikörper, einem Anti-Schilddrüsen-Peroxidase-Antikörper, einem Anti-Thyreoglobulin-Antikörper, einem Anti-Insulin-Antikörper, einem Anti-Ferritin-Antikörper, einem Anti-Alpha-Fetoprotein-Antikörper, einem Anti-Karzinoembryonalen Antikörper, einem Anti-Prostataspezifischen Antigen-Antikörper, einem antiluteinisierenden Hormon-Antikörper, einem Anti-Prolaktin-Antikörper, einem Anti-Human-Choriongonadotropin-Antikörper, einem Anti-Neuronspezifischen Enolase-Antikörper, einem Anti-Kohlenhydrat-Antigen-125-Antikörper, einem Antikohlenhydrat-Antigen-153-Antikörper, einem Anti-Kohlenhydrat-Antigen-199-Antikörper, einem Anti-Cytokeratin-Neunzehn-Fragment-Antikörper, einem Anti-Kohlenhydrat-Antigen-724-Antikörper, einem Anti -Kohlenhydrat-Antigen-242-Antikörper, einem Anti-Wachstumshormon-Antikörper, einem Anti-Myoglobin-Antikörper, einem Anti-Kohlenhydrat-Antigen-50-Antikörper, einem Anti-C-reaktiven Protein-Antikörper, einem Anti-Corticotropin-Antikörper, einem Anti-Kreatinkinase-Isoenzym-Antikörper, einem Anti-Sangtec-100-Protein-Antikörper, einem Anti-Laminin-Antikörper, einem Anti-Typ-IV-Kollagen-Antikörper, einem Antigehirn-natürlichen Peptid-N-terminalen Vorläufer-Protein-Antikörper, einem Anti-Troponin-Antikörper, einem Anti-Parathyroid-Hormon-Antikörper, einem Anti-Calcitonin-Antikörper, einem Anti-Procalcitonin-Antikörper, einem Anti-Prostata-Säure-Phosphatase-Antikörper, einem Anti-Osteocalcin-Antikörper, einem Anti-Schwangerschaftassoziierten Protein-A-Antikörper, einem Anti-Pepsinogen-I-Antikörper, einem Anti-Pepsinogen-II-Antikörper, einem Antiinsulin-ähnlichen Wachstumsfaktor-Antikörper oder einem Anti-D-Dimer-Antikörper ausgewählt ist.

20. Verwendung nach Anspruch 13, wobei das Kit zudem umfasst: die Reagenzien, die in der Expansionskultur von Zellkulturen verwendet werden, und die Herstellung und Reinigung des Zielantikörpers in großem Maßstab.

21. Verwendung nach Anspruch 13, wobei das Kit in einem halbautomatischen oder vollautomatischen Immunoassay-Analysator verwendet wird.

## Revendications

1. Procédé de préparation d'une paire d'anticorps, comprenant : utiliser un fragment de liaison à l'antigène d'un anticorps existant comme anticorps de capture et utiliser un anticorps à fragment anti-cristallisable comme un anticorps marqué pour dépister directement un anticorps cible qui peut être apparié au dit anticorps existant à partir du surnageant de culture cellulaire ; le fragment de liaison à l'antigène dudit anticorps existant est Fab' ou F(ab)₂ et ledit anticorps marqué est un anticorps anti-Fc.

2. Procédé selon la revendication 1, dans lequel le fragment de liaison à l'antigène dudit anticorps existant est F(ab')2 ;
comprenant de plus : optimiser les anticorps dépistés en fonction de la teneur totale en anticorps dans ledit surnageant de culture cellulaire ou de la force de liaison d'un anticorps à dépister dans ledit surnageant de culture cellulaire à un antigène cible, et ledit surnageant de culture cellulaire provient de cellules d'hybridome.

3. Procédé selon la revendication 1, dans lequel ledit anticorps marqué est marqué avec un marqueur-traceur, marquant directement ou indirectement ledit anticorps marqué.

4. Procédé selon la revendication 3, dans lequel ledit marqueur-traceur est choisi parmi au moins un marqueur enzymatique, un colorant fluorescent, un colorant chimiluminescent, l'isoluminol et ses dérivés et un marqueur radioactif.

5. Procédé selon la revendication 2, dans lequel ladite étape d'optimisation comprend :
a) détecter des anticorps dépistés pouvant être appariés avec l'anticorps de capture dans le surnageant de culture cellulaire par la méthode sandwich, et enregistrer le signal résultant en tant que OD₁ ;
b) détecter la teneur totale en anticorps dans ledit surnageant de culture cellulaire, et enregistrer le signal résultant en tant que OD₂ ;
c) détecter la force de liaison de l'anticorps à dépister dans ledit surnageant de culture cellulaire à l'antigène cible, et enregistrer le signal résultant en tant que OD₃ ; et
d) évaluer et optimiser l'anticorps dépisté dans ladite étape a) sur la base du rapport OD₁/OD₂ ou OD_{1/}OD₃.

6. Procédé selon la revendication 5, dans lequel ladite étape d) comprend :
1) si la culture cellulaire est une culture cellulaire à souche cellulaire unique, alors l'anticorps ayant un rapport OD₁/OD₂ plus élevé est mieux apparié au dit anticorps existant ;
2) si la culture cellulaire est une culture cellulaire à souches cellulaires multiples, alors la culture cellulaire ayant un rapport OD₁/OD₃ plus élevé est sélectionnée pour être séparée en cellules uniques pour une culture continue de manière à obtenir de nouvelles cultures cellulaires, et les nouvelles cultures cellulaires sont optimisées davantage à la suite de ladite étape 1).

7. Procédé selon la revendication 6, dans lequel ladite étape d) comprend spécifiquement :
diviser lesdites cultures cellulaires en deux groupes selon des puits de colonies uniques et des puits de colonies multiples ;
trier le groupe de puits de colonies uniques selon le rapport OD₁/OD₂ d'élevé à bas, et sélectionner les premières souches cellulaires N, dans lequel ledit N est 1-20 ; trier le groupe des puits de colonies multiples selon le rapport OD₁/OD₃ d'élevé à bas, et sélectionner les premiers puits de cellules M pour le sous-clonage, respectivement, puis détecter les cellules sous-clonées selon les procédés de ladite étape a) et de ladite étape b) et sélectionner les souches cellulaires N ayant le rapport OD₁/OD₂ plus élevé, dans lequel ledit M est 1-40.

8. Procédé selon la revendication 5, dans lequel ladite étape b) comprend spécifiquement : détecter la teneur totale en anticorps dans le surnageant de culture cellulaire en utilisant un anticorps se fixant spécifiquement à une zone d'anticorps conservée ou à une protéine de liaison d'immunoglobuline.

9. Procédé selon la revendication 8, dans lequel ledit anticorps se liant spécifiquement à une zone d'anticorps conservée est choisi parmi le mélange de deux ou plusieurs souches d'IgM de chèvre anti-souris, d'IgA de chèvre anti-souris, d'IgD de chèvre anti-souris, d'IgG1 de chèvre anti-souris, d'IgG2a de chèvre anti-souris, d'IgG2b de chèvre anti-souris, d'IgG3 de chèvre anti-souris, d'IgM de lapin anti-souris, d'IgA de lapin anti-souris, d'IgD de lapin anti-souris, d'IgG1 de lapin anti-souris, d'IgG2a de lapin anti-souris, d'IgG2b de lapin anti-souris et d'IgG3 de lapin anti-souris, et ladite protéine de liaison d'immunoglobuline est une protéine A de staphylocoque et/ou une protéine G de streptocoque.

10. Procédé selon la revendication 5, dans lequel ladite étape c) comprend spécifiquement : l'enrobage de l'antigène cible sur une phase solide, et la détection de la force de liaison de l'anticorps à dépister dans ledit surnageant de culture cellulaire à l'antigène cible au moyen d'un second anticorps.

11. Procédé selon la revendication 6, dans lequel ledit anticorps existant est un anticorps quelconque du groupe constitué par un anticorps anti-gastrine-17, un anticorps de protéine de liaison anti-folate, un anticorps anti-thyroperoxydase, un anticorps anti-thyroglobuline, un anticorps anti-insuline, un anticorps anti-ferritine, un anticorps anti-alpha-foetoprotéine, un anticorps anti-carcinoembryonnaire, un anticorps-antigène spécifique anti-prostate, un anticorps antihormone lutéinisante, un anticorps anti-prolactine, un anticorps anti-gonadotrophine chorionique humaine, un anticorps anti-énolase spécifique des neurones, un anticorps anti-antigène 125 des glucides, un anticorps anti-antigène 153 des glucides, un anticorps anti-antigène 199 des glucides, un anticorps anti-cytokératine à dix-neuf fragments, un anticorps anti-antigène 724 des glucides, un anticorps anti-antigène 242 des glucides, un anticorps antihormone de croissance, un anticorps anti-myoglobine, un anticorps anti-antigène 50 des glucides, un anticorps d'anti-protéine C réactive, un anticorps anti-corticotropine, un anticorps anti-créatine kinase isoenzyme, un anticorps anti-protéine Sangtec-100, un anticorps anti-laminine, un anticorps anti-collagène de type IV, un anticorps anti-protéine précurseur du peptide naturel N-terminal du cerveau, un anticorps anti-troponine, un anticorps antihormone parathyroïdienne, un anticorps anti-calcitonine, un anticorps anti-procalcitonine, un anticorps anti-prostate d'acide phosphatase, un anticorps anti-ostéocalcine, un anticorps anti-protéine A associée à la grossesse, un anticorps anti-pepsinogène I, un anticorps anti-pepsinogène II, un anticorps anti-facteur de croissance de type insuline ou un anticorps anti-D-dimère.

12. Procédé selon l'une quelconque des revendications 1 à 11, comprenant de plus l'expansion de la culture cellulaire contenant l'anticorps dépisté et la préparation et la purification à grande échelle de l'anticorps cible.

13. Utilisation d'un kit dans la préparation d'une paire d'anticorps, dans laquelle le kit comprend :
un anticorps de capture, étant un fragment de liaison à l'antigène d'un anticorps existant immobilisé sur un milieu en phase solide, le fragment de liaison à l'antigène dudit anticorps existant est Fab' ou F(ab)₂ ; et
un anticorps marqué étant un anticorps à fragment anti-cristallisable ; et
un antigène cible se liant directement ou indirectement à un milieu en phase solide ; et
un anticorps se liant spécifiquement à une zone d'anticorps conservée ou à une protéine de liaison d'immunoglobuline.

14. Utilisation selon la revendication 13, dans laquelle le fragment de liaison à l'antigène dudit anticorps existant est F(ab')₂.

15. Utilisation selon la revendication 13, dans laquelle ledit anticorps marqué est marqué avec un marqueur-traceur, marquant directement ou indirectement ledit anticorps marqué.

16. Utilisation selon la revendication 13, dans laquelle ledit marqueur-traceur est choisi parmi au moins un marqueur enzymatique, un colorant fluorescent, un colorant chimiluminescent, l'isoluminol et ses dérivés et un marqueur radioactif.

17. Utilisation selon la revendication 13, dans laquelle ledit anticorps se liant spécifiquement à une zone d'anticorps conservée est choisi parmi le mélange de deux ou plusieurs souches d'IgM de chèvre anti-souris, d'IgA de chèvre anti-souris, d'IgD de chèvre anti-souris, d'IgG1 de chèvre anti-souris, d'IgG2a de chèvre anti-souris et d'IgG2b de chèvre anti-souris, d'IgG3 de chèvre anti-souris, d'IgM de lapin anti-souris, d'IgA de lapin anti-souris, d'IgD de lapin anti-souris, d'IgG1 de lapin anti-souris, d'IgG2a de lapin anti-souris, d'IgG2b de lapin anti-souris et dIgG3 de lapin anti-souris, et ladite protéine de liaison d'immunoglobuline est une protéine A de staphylocoque et/ou une protéine G de streptocoque.

18. Utilisation selon la revendication 13, dans laquelle ladite phase solide est une plaque ELISA, des billes magnétiques ou de l'or colloïdal.

19. Utilisation selon la revendication 13, dans laquelle ledit anticorps existant est un anticorps quelconque du groupe constitué par un anticorps anti-gastrine-17, un anticorps de protéine de liaison anti-folate, un anticorps anti-thyroperoxydase, un anticorps anti-thyroglobuline, un anticorps anti-insuline, un anticorps anti-ferritine, un anticorps anti-alpha-foetoprotéine, un anticorps anti-carcinoembryonnaire, un anticorps-antigène spécifique anti-prostate, un anticorps antihormone lutéinisante, un anticorps anti-prolactine, un anticorps anti-gonadotrophine chorionique humaine, un anticorps anti-énolase spécifique des neurones, un anticorps anti-antigène 125 des glucides, un anticorps anti-antigène 153 des glucides, un anticorps anti-antigène 199 des glucides, un anticorps anti-cytokératine à dix-neuf fragments, un anticorps anti-antigène 724 des glucides, un anticorps anti-antigène 242 des glucides, un anticorps antihormone de croissance, un anticorps anti-myoglobine, un anticorps anti-antigène 50 des glucides, un anticorps d'anti-protéine C réactive, un anticorps anti-corticotropine, un anticorps anti-créatine kinase isoenzyme, un anticorps anti-protéine Sangtec-100, un anticorps anti-laminine, un anticorps anti-collagène de type IV, un anticorps anti-protéine précurseur du peptide naturel N-terminal du cerveau, un anticorps anti-troponine, un anticorps antihormone parathyroïdienne, un anticorps anti-calcitonine, un anticorps anti-procalcitonine, un anticorps anti-prostate d'acide phosphatase, un anticorps anti-ostéocalcine, un anticorps anti-protéine A associée à la grossesse, un anticorps anti-pepsinogène I, un anticorps anti-pepsinogène II, un anticorps anti-facteur de croissance de type insuline ou un anticorps anti-D-dimère.

20. Utilisation selon la revendication 13, dans laquelle ledit kit comprend de plus : les réactifs utilisés dans la culture d'expansion de cultures cellulaires et la préparation et la purification à grande échelle de l'anticorps cible.

21. Utilisation selon la revendication 13, dans laquelle ledit kit est utilisé dans un analyseur de dosage immunologique semi-automatique ou entièrement automatique.
